# NEUE EUROPÄISCHE PATENTSCHRIFT

(11) **EP 2 781 230 B2**
(45) Veröffentlichungstag und Bekanntmachung des Hinweises auf die Entscheidung über den Einspruch: **14.05.2025**
(45) Hinweis auf die Patenterteilung: 21.08.2019
(21) Anmeldenummer: 13160614.7
(22) Anmeldetag: 22.03.2013
(51) Int. Cl.: A61M 5/31, A61M 5/32, A61M 5/20, A61M 5/315

(54) **Substanzabgabevorrichtung mit Signalvorrichtung**
Substance dispensing device with a signaling device
Dispositif de distribution de substances avec dispositif de signal

(43) Veröffentlichungstag der Anmeldung: 24.09.2014
(60) Teilanmeldung: 19188038.4
(73) Patentinhaber: Ypsomed AG, 3401 Burgdorf (CH)
(72) Erfinder: Tschirren, Markus, 3400 Burgdorf (CH); Hirschel, Jürg, 3007 Bern (CH)
(74) Vertreter: Meier Obertüfer, Jürg

(56) Entgegenhaltungen:
- WO-A1-2011/043714
- WO-A1-2012/117255
- US-A1- 2005 222 539
- US-A1- 2010 137 798

## Beschreibung

Die Erfindung betrifft eine Substanzabgabevorrichtung, insbesondere eine Injektionsvorrichtung oder einen Autoinjektor, die eine Signalisierungsvorrichtung aufweist, um z. B. akustisch und/oder taktil anzuzeigen, wann eine Abgabe einer Substanz oder eine Ausschüttung vollständig oder mindestens zu einem vorgegebenen Teil erfolgt ist. Eine z.B. automatisch ausschüttbare Substanz kann ein fluides Produkt oder Medikament sein, welches z.B. flüssig, pastenartig oder gelartig ist.

Aus der WO 2011/123024 A1 ist eine Medikamentenabgabevorrichtung mit einer auf einen Medikamentenbehälter wirkenden Antriebseinrichtung zum Ausstoßen eines Medikaments bekannt, wobei eine Halteeinrichtung konfiguriert ist, um die Antriebseinrichtung in einem vorgespannten Zustand zu halten. Eine Aktivierungseinrichtung wirkt mit der Halteeinrichtung zum Lösen der Antriebseinrichtung aus dem vorgespannten Zustand zusammen. Eine Rückmeldeeinrichtung kann sowohl mit der Halteeinrichtung als auch mit der Antriebseinrichtung zusammenwirken, um ein Signal zu erzeugen, das anzeigt, dass das Medikament vollständig ausgestoßen wurde.

Die WO 94/11041 offenbart einen Auto-Injektor mit einer ersten Einheit, welche eine automatische Nadelpenetration bewirkt und welche eine zweite Einheit steuert, welche die Medikamentenabgabe bewirkt, so dass die Medikamentenabgabe nur dann begonnen wird, wenn die Nadelpenetration vollständig durchgeführt wurde.

Die WO 2013/016832 A1 offenbart eine Injektionsvorrichtung zum automatischen Ausschütten mit einer Nadelschutzeinrichtung, die von einer distalen in eine proximale Position und von dieser in eine Nadelschutzposition verschiebbar ist und mit einer im Gehäuse bewegbaren Antriebseinrichtung, welche durch ein Antriebsmittel in eine Ausschüttposition getrieben wird und mit einer Drehhülse, die von einer ersten in eine zweite Position drehbar ist.

In der US20100127798 ist ein Autoinjektor beschrieben, bei dem nach der Injektion des Medikaments der Spritzenhalter (10) mit der integrierten Spritze (2) nach der Injektion zurückgezogen wird. Der sogenannte Spritzenrückzug geschieht mittels der Rückzugsfeder (7). Wobei nach der Injektion des Medikaments und kurz vor dem Spritzenrückzug ein Signal erzeugt wird. Hierfür verschiebt die Rückzugsfeder (7) für die Signalerzeugung den Spritzenhalter (10) nach proximal um den proximalen Bereich (switch cam 17) vom Spritzenhalter (10) gegen eine Fensteröffnung (20) anzuschlagen und so ein Signal zu generieren. Nach dem Signal wird der Spritzenhalter (10) von der Rückzugsfeder (7) vollständig zurückgezogen, damit die Nadel (4) aus der Injektionsstelle herausgezogen wird.

WO2013/016832 A1 weist ein erstes Profil auf, welches in Wirkverbindung mit einem zweiten Profil der Antriebseinrichtung steht. Das Antriebsmittel dreht durch das erste Profil und das zweite Profil die Drehhülse von der ersten in die zweite Position. US 20100137798 offenbart einen Autoinjektor, bei dem nach der Injektion des Medikaments ein Spritzenhalter mit der integrierten Spritze zurückgezogen wird. Der sogenannte Spritzenrückzug geschieht mittels der Rückzugsfeder, wobei nach der Injektion des Medikaments und kurz vor dem Spritzenrückzug ein Signal erzeugt wird. Hierfür verschiebt die Rückzugsfeder den Spritzenhalter nach proximal gegen eine Fensteröffnung einer Betätigungshülse. Nach dem Signal wird der Spritzenhalter von der Rückzugsfeder vollständig zurückgezogen und die Nadel aus der Injektionsstelle herausgezogen.

Es ist eine Aufgabe der Erfindung eine Substanzabgabevorrichtung oder Injektionsvorrichtung bereitzustellen, bei welcher der Anwender auf einfache Art eine Injektion auslösen und durch die Vorrichtung über den korrekten Funktionsablauf informiert werden kann. Diese Aufgabe wird gelöst durch eine Vorrichtung mit den Merkmalen des unabhängigen Anspruchs.

Eine erfindungsgemäße Vorrichtung zum Verabreichen einer Substanz ist in Anspruch 1 definiert und umfasst eine Injektionsfeder, mit welcher die Ausschüttung automatisch durchgeführt werden kann. Dabei ist keine extern z.B. von einem Benutzer zuzuführende oder aufzuwendende Kraft oder Energie erforderlich. Die Injektionsfeder speichert vorteilhaft die vollständige für eine automatische Substanzabgabe erforderliche Energie. Diese Feder kann in einem energiespeichemden Zustand, also z.B. komprimiert, auseinandergezogen oder auch verdreht, in die Injektionsvorrichtung eingebaut werden und durch einen Energieabgabevorgang, also z. B. durch Entspannen, wenn die Feder komprimiert oder verdreht bzw. auf Torsion beansprucht eingebaut wurde, oder auch durch Zusammenziehen, wenn die Feder auseinandergezogen eingebaut wurde, Energie abgeben. Die Energieabgabe erfolgt vorteilhaft unmittelbar oder mittelbar, d. h. über zwischengeschaltete Bauelemente, an eine Kolbenstange oder ein Druckelement, welches auf einen Stopfen einer Spritze drückt und diesen Stopfen in die Spritze einschieben kann.

Optional kann das Energiespeicherelement oder ein weiteres separates Energiespeicherelement vorgesehen sein, um auch den Vorgang des Einstechens einer Nadel zu automatisieren. Der Einstechvorgang kann jedoch auch manuell erfolgen, also z. B. durch einen Benutzer vorgenommen werden, ohne hierfür in der Injektionsvorrichtung gespeicherte Energie zu verwenden.

Die Verabreichungsvorrichtung weist eine Nadel als Abgabeelement auf, durch welches die Substanz abgegeben werden kann. Die Nadel ist auf bekannte Art mit dem Behältnis für die abzugebende Substanz gekoppelt, sodass z. B. beim Verschieben des oben erwähnten Stopfens die Substanz durch die Nadel hindurchtritt und am distalen vorderen Ende der Substanz abgegeben und injiziert wird.

Für das Abgabeelement ist ein Schutzelement vorgesehen, beispielsweise eine über das Abgabeelement hinauszuschiebende Hülse, welche beispielsweise parallel zur Längsrichtung einer als Abgabeelement dienenden Nadel axial verschoben werden kann. Das Schutzelement kann vor der Abgabe das Abgabeelement z. B. radial umgeben und auch distal über das Abgabeelement hinausragen, so dass das Abgabeelement im Wesentlichen oder vollständig durch das Schutzelement umgeben oder bedeckt wird. Dabei weist das Schutzelement vorteilhaft eine Durchtrittsöffnung für das Abgabeelement auf, durch welche das Abgabeelement den Schutzbereich des Schutzelements verlassen kann. Es kann sowohl das Abgabeelement aktiv durch das Schutzelement hindurchbewegt werden als auch das Schutzelement aktiv von dem Abgabeelement entfernt, also z. B. in proximale Richtung zurückgeschoben, werden, um zumindest den distalen Bereich des Abgabeelements freizugeben.

Das Schutzelement ist mit einem Antriebselement gekoppelt, welches separat von den oben erwähnten optional vorgesehenen Antriebselementen ausgebildet ist, z. B. als eine zweite oder dritte Antriebsfeder innerhalb einer Injektionsvorrichtung. Mit diesem Antriebselement kann das Schutzelement angetrieben werden. Z. B. kann das Antriebselement bewirken, dass das Schutzelement nach erfolgter Substanzabgabe wieder über das Abgabeelement gebracht oder geschoben werden kann. Optional kann das Antriebselement auch als Halteelement dienen, z. B. um das Schutzelement vor und/oder nach einer Substanzabgabe in einer Schutzstellung über oder um das Abgabeelement zu halten. Das Antriebselement kann in einem entspannten Zustand oder in einem gespannten oder geladenen, also Energie beinhaltenden oder speichernden, Zustand in der Injektionsvorrichtung eingebaut sein, wobei diese Energie zum Antreiben des Schutzelements dienen kann. Wird das Antriebselement in einem Zustand in der Injektionsvorrichtung eingebaut, in welchem dieses keine oder wenig Energie speichert, so muss das Antriebselement während eines Funktionsablaufes vor dem Antreiben des Schutzelements von einem anderen Element mit Energie versorgt werden, also von einem der erwähnten weiteren Antriebselemente, z.B. einer gespannten Ausschüttfeder.

Eine Rückkopplungsvorrichtung der Injektionsvorrichtung erzeugt ein Signal, wenn eine vorgegebene oder die gesamte Menge der abzugebenden Substanz abgegeben wurde. Das erzeugte Signal kann ein akustisches Signal sein, also z. B. ein "Klick"-Geräusch, welches erzeugt wird, wenn ein sich bewegendes Element an einem anderen anschlägt. Das Signal kann auch ein taktiles oder haptisches Signal sein, also ein Signal, welches von einem Benutzer erfühlt werden kann. Ein solches Signal kann z. B. ebenfalls durch einen Anschlag eines Elements an einem anderen Element erzeugt werden.

Erfindungsgemäß ist die Rückkopplungsvorrichtung mit dem Antriebselement gekoppelt, mit welchem auch das Schutzelement gekoppelt ist. Das Rückkopplungsvorrichtung ist ein Anschlagelement, also ein Pin oder eine Hülse, welche von einer Nadelschutzhülsenfeder beschleunigt oder gegen einen Anschlag bewegt wird, wobei die Nadelschutzhülsenfeder ein Nadelschutzelement mit Druck beaufschlagt oder Kraft auf dieses ausübt, um das Nadelschutzelement relativ zur Nadel in eine Schutzposition zu bringen oder zu halten.

Das Rückkopplungselement kann z. B. an dem Gehäuse der Injektionsvorrichtung anschlagen, ist also relativ zu diesem bewegbar, wobei es auch möglich ist, dass das Rückkopplungselement an jedem anderen Teil anschlägt. Vorzugsweise wird das Rückkopplungselement von dem erwähnten Antriebselement über eine vorgegebene Strecke z. B. geradlinig oder in einer Drehbewegung beschleunigt, um mit einer bestimmten Geschwindigkeit einen Aufschlag und somit ein bemerkbares Rückkopplungssignal zu erzeugen.

Die erfindungsgemäße Vorrichtung kann eine weitere separate zweite Rückkopplungsvorrichtung aufweisen, welche z. B. den Beginn der Substanzabgabe signalisiert. Es ist denkbar, dass beide Rückkopplungsvorrichtungen auch in einem Element realisiert werden können.

Wie bereits erwähnt, weist die Verabreichungsvorrichtung auch ein weiteres Antriebselement oder Energiespeicherelement auf, welches Energie zur Bewirkung der Substanzabgabe und/oder Energie zur Durchführung eines Nadeleinstichs zur Verfügung stellt. Dieses weitere Antriebselement ist separat von dem Antriebselement vorgesehen, welches mit der Rückkopplungsvorrichtung gekoppelt ist und kann z. B. funktional vollständig von dem Rückkopplungs-Antriebselement getrennt sein. Ebenso ist es möglich, dass ein solches weiteres Antriebselement Energie an das Rückkopplungs-Antriebselement abgibt oder überträgt.

An dem Rückkopplungselement kann auch ein Modulations- oder Dämpfungselement vorgesehen sein, mittels welchem die Rückkopplung, also z. B. ein Rücckopplungsgeräusch oder ein taktiles Rückkopplungssignal, beeinflusst oder modifiziert werden kann. Beispielsweise können Laschen oder Anschlagflächen oder Dämpfungsmittel vorgesehen sein, welche einen Aufprall oder Aufschlag der Rückkopplungsvorrichtung auf einer Fläche oder einem Aufschlagsteil verzögern oder abbremsen. Ebenso ist es möglich, dass ein Modulationselement die Anschlagsfläche vergrößert, um die zu erzeugende Signalstärke zu vergrößern.

Das Rückkopplungselement kann vorzugsweise von einer lösbaren Haltevorrichtung bis zum Ende der Substanzabgabe gehalten werden, sodass beispielsweise das Rückkopplungselement erst dann freigegeben oder ausgelöst und z. B. erst dann von einem Antriebselement angetrieben wird, wenn die Substanzabgabe vollständig oder bis zu einem gewissen vorher definierten Grad erfolgt ist, also z. B. die Kolbenstange bis zu einem festgelegten Ausschüttpunkt verschoben wurde.

Die Rückkopplungsvorrichtung ist ein Anschlagselement, welches entlang einer geraden Strecke beschleunigt oder angetrieben wird, um am Ende an einem Anschlagsort aufzutreffen und so ein Rückkopplungssignal zu erzeugen. Ebenso ist es möglich, dass die Rückkopplungsvorrichtung beispielsweise ein Drehelement ist, welches z. B. nach der festgestellten Beendigung der vollständigen Substanzabgabe eine Drehbewegung ausführt und einen Drehanschlag erzeugt. Hierfür kann z. B. ein Torsionsmoment einer verwendeten Feder, also z. B. einer verwendeten Nadelschutzfeder oder auch einer Injektionsfeder, verwendet werden.

Gemäß einem weiteren Aspekt bezieht sich die Erfindung auf ein Verfahren zum Abgeben einer Substanz aus einer wie oben beschriebenen Vorrichtung, wobei die Substanz mittels eines ersten Antriebselements automatisch abgegeben wird und ein Rückkopplungssignal zur Signalisierung der z. B. beendeten Substanzabgabe mittels eines von einem separaten zweiten Antriebselements angetriebenen Rückkopplungselements erzeugt wird. Die Antriebselemente sind folglich separate Vorrichtungen oder Funktionseinheiten, also beispielsweise zwei getrennt vorgesehene Federn, welche jedoch miteinander gekoppelt werden können, um Energie von einer Feder, also z. B. von einer Antriebsfeder, auf eine andere Feder, also z. B. die Rückkopplungsfeder, zu übertragen.

Diese Energie- oder Kraftübertragung erfolgt während der Substanzabgabe.

Die Erfindung wird nur anhand der ersten Ausführungsform beschrieben. Es zeigen:
- Fig. 1: eine Querschnittsansicht entlang einer Längsachse einer ersten Ausführungsform einer Injektionsvorrichtung zur Veranschaulichung des Konzeptaufbaus;
- Fig. 2: eine Explosionsansicht der in Fig. 1 gezeigten Injektionsvorrichtung;
- Fig. 3A und 3B: um 90° zueinander gedrehte Querschnittsansichten der Injektionsvorrichtung im Auslieferungszustand;
- Fig. 3C bis 3G: Abwicklung der radialen Innenseite der Verriegelungshülse (180° Abwicklung) zur Veranschaulichung der Relativbewegung der eingreifenden Nocken des Mechanikhalters und des Klick Pins
- Fig. 4A und 4B: um 90° gedrehte Querschnittsansichten der Injektionsvorrichtung im eingestochenen Zustand, wenn die Injektionsvorrichtung auf die Injektionsstelle gedrückt wird oder wurde;
- Fig. 4D bis 4H: Detailansicht der Verriegelungshülse und das Prinzip der Nadelschutzverriegelung zur Veranschaulichung der Relativbewegung der eingreifenden Nocken des Mechanikhalters und des Klick Pins
- Fig. 5A und 5B: um 90° zueinander gedrehte Querschnittsansichten der Injektionsvorrichtung vor dem Beginn der Ausschüttung;
- Fig. 6A und 6B: um 90° zueinander versetzte Querschnittsansichten der Injektionsvorrichtung nach erfolgter Ausschüttung;
- Fig. 7A und 7B: um 90° zueinander versetzte Querschnittsansichten der Injektionsvorrichtung nach erfolgter Ausschüttung und diese erfolgte Ausschüttung bestätigenden Anschlag eines Klick-Elements;
- Fig. 8: eine Explosionsansicht einer zweiten Ausführungsform einer Injektionsvorrichtung
- Fig. 9A und 9B: um 90° zueinander gedrehte Querschnittsansichten der Injektionsvorrichtung im Auslieferungszustand;
- Fig. 10A und 10B: um 90° zueinander versetzte Längsquerschnittsansichten der Injektionsvorrichtung im eingestochenen Zustand, wenn die Injektionsvorrichtung auf die Injektionsstelle gedrückt wurde;
- Fig. 11A und 11B: um 90° zueinander versetzte Querschnittsansichten der Injektionsvorrichtung nach erfolgter Ausschüttung und erfolger Signalisierung des Endes der Injektion durch Anschlag der Freigabehülse an der Endkappe;
- Fig. 12A und 12B: eine um 90° zueinander gedrehte Querschnittsansichten einer dritten Ausführungsform einer Injektionsvorrichtung im Auslieferungszustand;
- Fig. 13A und 13B: um 90° zueinander versetzte Querschnittsansichten der Injektionsvorrichtung nach erfolgter Einstech- und Ausschüttbewegung und Bestätigung der vollständigen Ausschüttung durch Anschlag der Freigabehülse am Boden des Mechanikhalters.
- Fig. 14A: zeigt eine weitere Ausführungsform einer Injektionsvorrichtung im Auslieferungszustand, bei welcher die Spritzenfeder die Energie für den Endklick (End-of-Injection-Klick) zur Verfügung stellt;
- Fig. 14B: zeigt die Injektionsvorrichtung gemäß Fig. 14A im ausgeschütteten Zustand vor dem Endklick;
- Fig. 15A: zeigt eine weitere Ausführungsform einer End-Klick-Einheit im Auslieferungszustand, bei welcher ein rotativer Anschlag durch ein Torsionsmoment der Injektionsfeder erzeugt wird;
- Fig. 15B: zeigt die Funktionseinheit von Fig. 15A bei erzeugtem Endklick;
- Fig. 16A: zeigt eine weitere Ausführungsform einer Funktionseinheit im Auslieferungszustand zur Erzeugung eines Endklicks durch die Energie der Nadelschutzhülsenfeder;
- Fig. 16B: zeigt die Funktionseinheit von Fig. 16A nach erfolgtem Endklick.

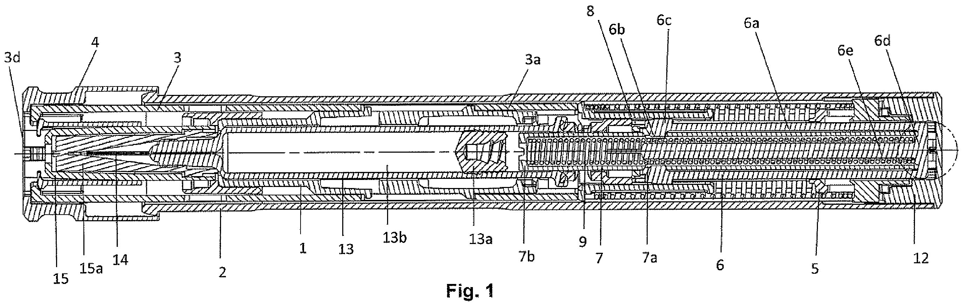

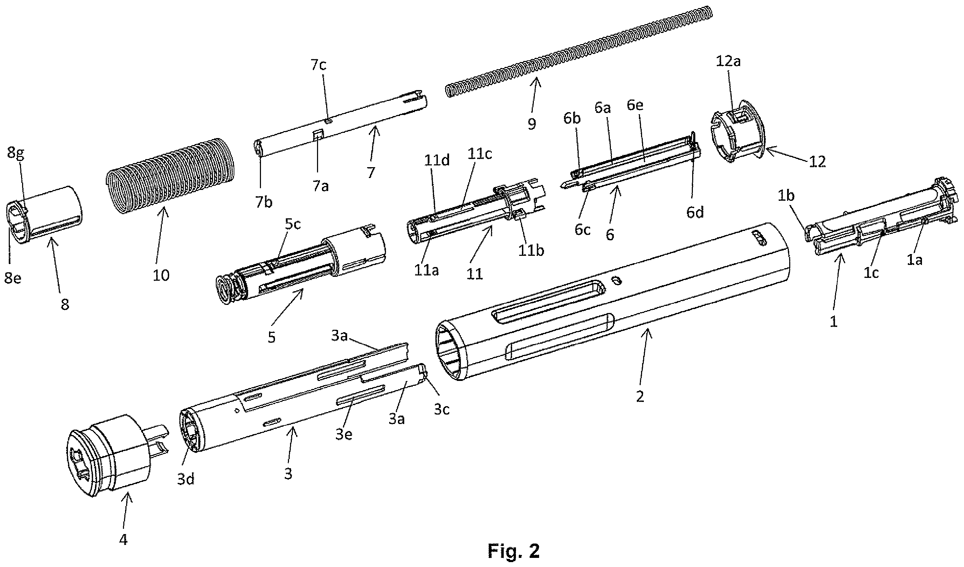

Die Figuren 1 und 2 zeigen einen Konzeptaufbau einer ersten Ausführungsform einer erfindungsgemäßen Injektionsvorrichtung. Die Injektionsvorrichtung umfasst ein hülsenförmiges Gehäuse 2, an welchem ein Spritzenhalter 1, ein Mechanikhalter 5 und eine Endkappe 12 gehäusefest angeordnet, also bezüglich des Gehäuses 2 unbeweglich sind. Der Spritzenhalter 1, der Mechanikhalter 5 und die Endkappe 12 können mit dem Gehäuse 2 verrastet, verklebt, verschweist, verriegelt oder verschnappt oder auch jeweils oder insgesamt einteilig mit dem Gehäuse 2 ausgebildet sein. In dem Spritzenhalter 1 kann eine vorzugsweise bereits vorgefüllte Spritze 13 aufgenommen und von diesem gehalten werden. Die Spritze 13 weist einen durch einen entlang der Längsachse der Spritze 13 verschiebbaren Stopfen 13a begrenzten Aufnahmeraum 13b auf, in welchem eine abzugebende Substanz enthalten ist, welche durch Verschiebung des Stopfens 13a in distale Längsrichtung der Spritze 13 (in Fig. 1 nach links) aus diesem Raum heraus verdrängt und durch eine an der Vorderseite der Spritze 13 angeordnete Nadel 14 auf bekannte Art abgegeben werden kann. Die Nadel 14 ist im Fig. 1 gezeigten Ausgangszustand von einer Nadelschutzkappe 15 umgeben, in welcher ein z. B. elastisches oder aus Gummi gefertigtes Nadelschutzelement 15a vorhanden ist. Die Nadelschutzkappe 15 kann zusammen mit dem darin enthaltenen Gummielement 15a mittels des darauf aufgesetzten Kappenabzugselements 4 abgezogen werden, um die Nadel 14 freizulegen, welche in der gezeigten Ausgangslage auch von der Vorderseite einer in axialer Richtung des Gehäuses 2 einschiebbaren und wieder ausfahrbarer Nadelschutzhülse 3 umgeben wird.

Eine als Energiespeicher oder Antriebsmittel dienende Injektionsfeder 9 ist vorgespannt und wird im Ausgangszustand zwischen der Kolbenstange 7 und dem Klick-Pin 6 gehalten, wobei die Injektionsfeder 9 innerhalb der Kolbenstange 7 gelagert ist und von der Kolbenstange 7 umgeben wird und auf ein distales Bodenelement 7b der Kolbenstange 7 drückt bzw. sich gegen dieses abstützt. In proximaler Richtung wird die Injektionsfeder 9 von einem proximalen Boden- oder Tellerelement 6d eines Klick-Pins 6 gehalten bzw. stützt sich gegen dieses ab. Der Klick-Pin 6 weist einen in axialer Richtung verlaufenden Mittelsteg 6e auf, welcher im eingesetzten Zustand innerhalb der Injektionsfeder 9 angeordnet ist und am proximalen Ende mit dem Tellerelement 6d verbunden ist, von welchem sich in etwa parallel verlaufend zum Mittelsteg 6e Auslöseschnapparme 6a erstrecken, welche verformbar oder federnd ausgebildet sind, so dass die an jedem Auslöseschnapparm 6a angeordneten radial nach innen vorstehenden Innennocken 6b und radial nach außen vorstehenden Außennocken 6c in radialer Richtung, also radial nach außen oder innen bewegt werden können. Die Innennocken 6b greifen in Vertiefungen oder Öffnungen 7a der Kolbenstange 7 ein, so dass in diesem Zustand eine axiale Verschiebung zwischen Klick-Pin 6 und Kolbenstange 7 durch die Kraft der vorgespannten Injektionsfeder 9 nicht möglich ist. Das aus Klick-Pin 6, Kolbenstange 7 und Injektionsfeder 9 bestehende Federpaket kann somit nicht auseinander gedrückt werden. Ein radiales Ausweichen der Auslöseschnapparme 6a nach außen wird durch die im Bereich der Außennocken 6c um den Klick-Pin 6 herum angeordneten axial verschiebbare Verriegelungshülse 8 verhindert, deren Innenseite die Außennocken 6c gegenüberliegen oder an dieser anliegen.

Die Injektionsfeder 9 ist vorzugsweise eine Druckfeder oder Wendelfeder, welche bevorzugt mindestens die Energie für eine Ausschüttsequenz speichert oder aufnehmen kann und welche als so gespannte Feder in die Injektionsvorrichtung eingesetzt wird.

Die Nadelschutzeinrichtung 3, welche als hülsenförmiges Element ausgebildet ist und relativ zum Gehäuse 2 verschiebbar gelagert ist, weist an ihrer distalen Stirnseite eine Durchtrittsöffnung 3d auf, durch welche die Nadel 14 hindurchtreten bzw. welche in axialer Richtung entlang der Nadel 14 zurück bewegt oder in das Gehäuse 2 eingeschoben werden kann. In axialer Richtung sich erstreckend sind zwei gegenüberliegende Stege 3a vorgesehen, welche sich relativ zur Durchtrittsöffnung 3d in proximaler Richtung befinden.

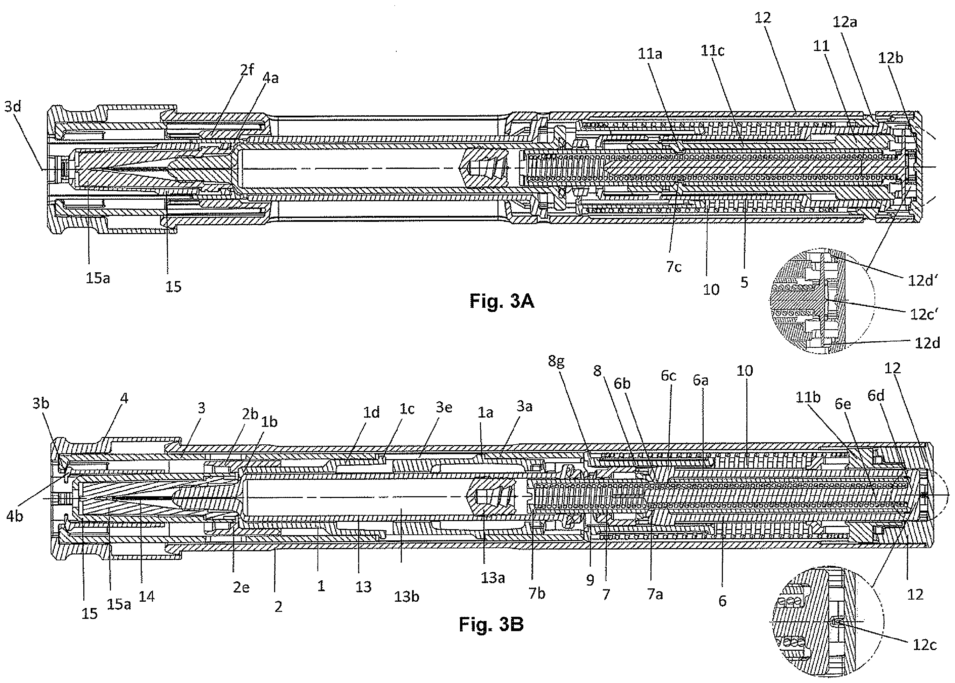

Die Figuren 3A und 3B sind relativ zueinander um 90° versetzte Querschnittsansichten der Injektionsvorrichtung im Auslieferungszustand. Wie bereits erwähnt, ist die Injektionsfeder 9 zwischen der Kolbenstange 7 und dem Klick-Pin 6 vorgespannt. Die Kolbenstange 7 wird gegen die Kraft der Injektionsfeder 9, welche eine auf die Kolbenstange 7 in distaler Richtung wirkende Kraft ausübt, durch die Innennocken 6b der Auslöseschnapparme 6a des Klick-Pins 6 gehalten, wie aus Fig. 3B ersichtlich. Die Verriegelungshülse 8 verhindert eine radial nach außen gerichtete Bewegung der Nocken 6b und der Auslöseschnapparme 6a.

Ein End-Klick-Element 11 wird durch eine Nadelschutzhülsenfeder 10, welche auf eine ringförmige radiale Aufweitung 11b des Klick-Elements 11 drückt, in proximale Richtung gedrückt bzw. gehalten. An der gegenüberliegenden Seite stützt sich die Nadelschutzhülsenfeder 10 an einem Flansch 8g der Verriegelungshülse 8 ab. Die Feder 10 kann ebenso wie die Feder 9 bereits vorgespannt oder auch entspannt in die Injektionsvorrichtung eingesetzt werden. Die Verriegelungshülse 8 liegt proximal versetzt an den Stegen 3a der Nadelschutzhülse 3 an, welche über radial nach außen stehende in Ausnehmungen oder Durchgänge der Stege 3a eingreifende proximale Nocken 1a des gehäusefesten Spritzenhalters 1 gegen eine Verschiebung in distale Richtung gehalten wird.

Das so nach proximal gedrückte oder gehaltene Klick-Element 11 hält, wie in Fig. 3A gezeigt, mittels radial nach innen vorstehender auf Federarmen 11c angeordneter Nocken 11a, welche in Vertiefungen oder Öffnungen 7c der Kolbenstange 7 eingreifen, die Kolbenstange 7 in der gezeigten proximalen Ausgangsposition, bei welcher die distale Stirnseite der Kolbenstange 7 von der proximalen Rückseite des Stopfens 13a beabstandet ist.

Das Klick-Element 11 liegt im Ausgangs- oder Auslieferzustand mit seiner proximalen Stirnfläche auf dem tellerförmigen Boden 12b der mit dem Gehäuse 2 mittels der Schnapper 12a verschnappten oder gehäusefesten Endkappe 12 auf.

Die in die Injektionsvorrichtung eingesetzte Spritze 13 wird im Spritzenhalter 1 mittels einer Schulterauflage 1b oder radial nach innen vorstehenden Vorsprüngen 1b des Spritzenhalters 1 nach vorne gehalten und mittels eines Ringes oder einer Gehäuseverjüngung 2b im Gehäuse 2 gesichert. Dieser Ring bzw. die Gehäuseverjüngung 2b verhindert ein radiales Ausweichen der Spritzenhalterung 1b. Der Spritzenhalter 1 liegt stirnseitig auf radial nach innen vorstehenden Rippen oder Vorsprüngen 2e des Gehäuses 2 auf.

Radial nach außen vorstehende Nocken 1c auf Federarmen 1d des gehäusefesten Spritzenhalters 1 greifen in eine axial verlaufende Nut 3e eines jeden Stegs 3a der Nadelschutzhülse 3 ein und sind von dem oben erwähnten radial vorstehenden Nocken 1a in axialer Richtung so weit wie etwa die Länge der axialen Nut 3e beabstandet, so dass die Nadelschutzhülse 3 durch die in die axiale Nut 3e eingreifenden axial voneinander beabstandeten Nocken 1a und 1c gegen axiale Verschiebung gehalten wird, wobei die auf den Federarmen 1d vorgesehenen Nocken 1c bei Anliegen einer relativ kleinen Eindrückkraft an der Nadelschutzhülse 3 radial nach innen ausweichen und ein proximales Einschieben der Nadelschutzhülse 3 in das Gehäuse ermöglichen.

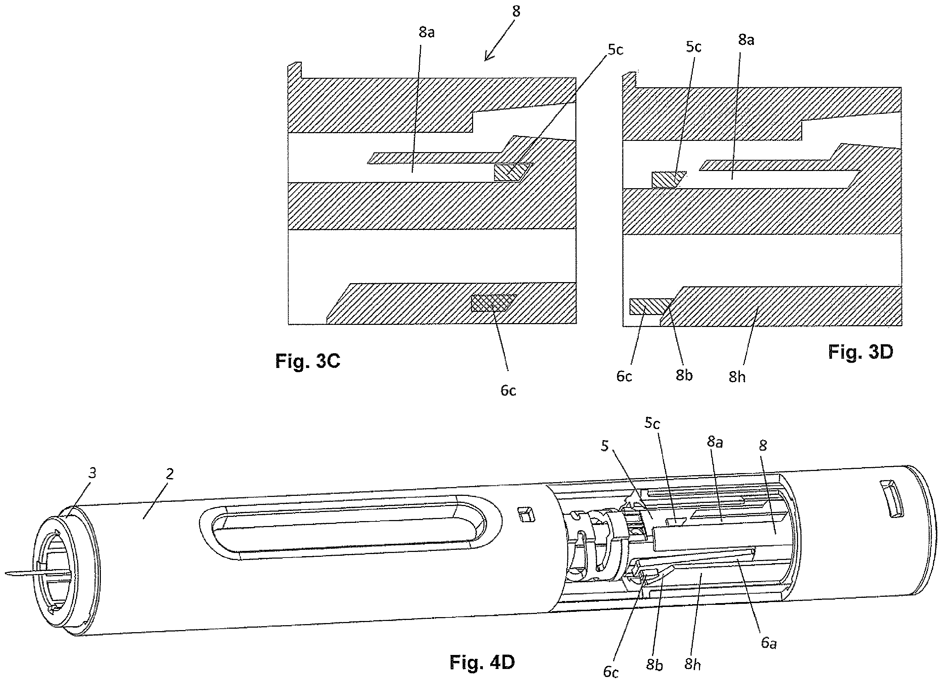

Die Fig. 3C - 3G zeigen eine Innenabwicklung von 180°, d. h. einer Hälfte, der Verriegelungshülse 8, wobei radial nach innen vorstehende Bereiche schraffiert dargestellt sind. Es werden dabei die Positionen der Verriegelungshülse 8 in Verbindung mit den Bewegungen des Nadelschutzes 3 erläutert.

In der Fig. 3C wird die Verriegelungshülse im Ausgangszustand des Nadelschutzes gezeigt. Die zwischen den schraffierten Bereichen liegenden Führungsbereiche oder Nuten der Verriegelungshülse 8 ermöglichen einen Eingriff einer radial nach außen gerichteten Nocke 6c des Klickpins 6. Weiterhin greift ein in radiale Richtung nach außen vorstehender Nocken 5c des Mechanikhalters 5 in eine auf der radialen Innenseite der Verriegelungshülse 8 vorgesehene axial verlaufende Nut 8a hinein und ist mit dieser im Eingriff, so dass ein Verdrehen der Verriegelungshülse 8 relativ zum gehäusefesten Mechanikhalter 5 verhindert wird. Die Verriegelungshülse 8 kann in der gezeigten Position jedoch in axiale Richtung verschoben werden.

Vor Beginn der Benutzung der Injektionsvorrichtung muss die Nadel 14 freigelegt werden, wozu das Kappenabzugselement 4 in distaler Richtung von der distalen Vorderseite der Injektionsvorrichtung abgezogen wird. Wie aus Fig. 3A ersichtlich, schnappen die Schnapphaken 4a des Kappenabzugselements 4 hinter dem hinteren Rand der Nadelschutzkappe 15 ein. Auf der radial äußeren Seite der Schnapphaken 4a vorgesehene an diesen anliegende Rippen 2f des Gehäuses 2 verhindert ein Ausweichen dieser Schnapphaken 4a, so dass durch ein Ziehen an dem Kappenabzugselement 4 die Nadelschutzkappe 15 in distaler Richtung abgezogen werden kann. Dabei wird auch der innerhalb der Nadelschutzkappe 15 befestigte elastische oder Gumminadelschutz 15a zusammen mit der Nadelschutzkappe 15 abgezogen, so dass die Nadel 14 freigelegt wird. Das Kappenabzugselement 4 wird mittels eines Schnappelements 4b, welches hinter eine radial vorstehende Schnapphalterung 3b eingreift, an der Nadelschutzhülse 3 gehalten. Beim Abziehen des Kappenabzugselements 4 muss eine Kraft aufgewendet werden, welche die Schnapphalterung 3b, 4b überwinden kann.

Nach Abnahme des Kappenabzugselements 4 zusammen mit der Nadelschutzkappe 15 liegt die Nadel 14 frei, wird jedoch noch von dem distalen Hülsenbereich der Nadelschutzhülse 3 umgeben, welcher auch in distale Richtung über die Spitze der Nadel 14 hinausragt, so dass die Nadel 14 durch den vorderen oder distalen Teil der Nadelschutzhülse 3 noch geschützt wird.

Wird die sich nach dem Abnehmen des Kappenabzugselements 4 im einsatzbereiten Zustand befindliche Injektionsvorrichtung mit ihrer distalen Stirnseite, also dem distalen vorderen Bereich der Nadelschutzhülse 3, auf eine Einstichstelle aufgedrückt, so verschiebt sich durch diesen üblicherweise von einem das Gehäuse 2 haltenden Benutzer auf das Gehäuse 2 aufgebrachten Druck die Nadelschutzhülse 3 in proximaler axialer Richtung in das Gehäuse 2 hinein, wodurch die relativ zum Gehäuse 2 feststehende Nadel 14 freigelegt und in die Einstichstelle eingestochen wird.

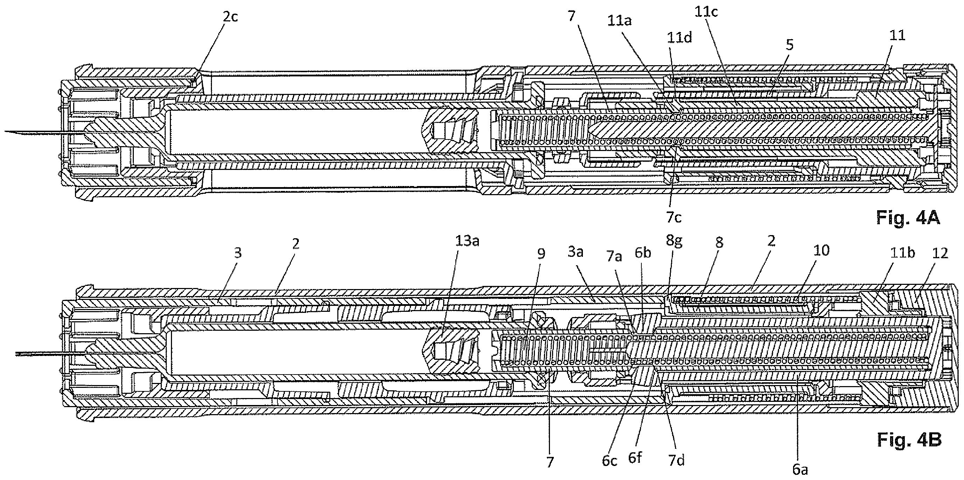

Dieser Zustand ist in den um 90° zueinander gedreht versetzten Querschnittsansichten der Figuren 4A und 4B gezeigt. Die Nadelschutzhülse 3 wird bei entsprechendem auf das Gehäuse 2 in distale Richtung ausgeübten Druck durch die die Einstichstelle umgebende Auflagefläche in das Gehäuse 2 bis zum den Einschub der Nadelschutzhülse 3 begrenzenden Anschlag 2c eingeschoben, was zeitgleich mit dem Einstechen der Nadel 14 in die Injektionsstelle erfolgt.

Durch das Einschieben der Nadelschutzhülse 3 wird die, wie in Fig. 3B und 4B gezeigt, an den proximalen Ende der Stege 3a der Nadelschutzhülse 3 anliegende Verriegelungshülse 8 ebenfalls in proximale Richtung relativ zum Gehäuse 2 verschoben, wodurch die Nadelschutzhülsenfeder 10, welche sich zwischen den erwähnten Anlageflächen der Verriegelungshülse 8 und dem Klick-Element 11 abstützt, komprimiert oder gestaucht wird.

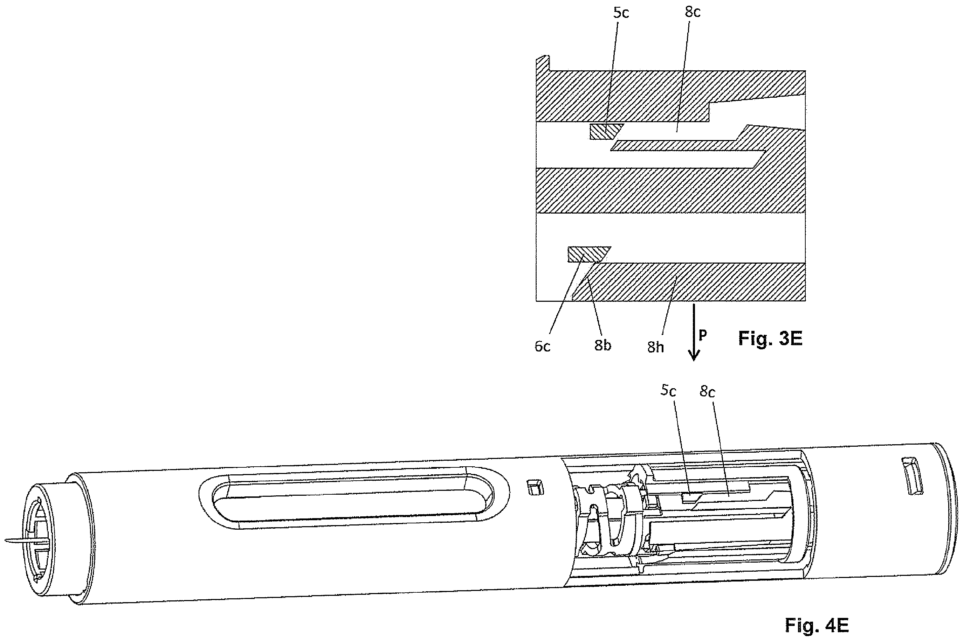

Der auf einem elastischen Auslöseschnapparm 6a des Klickpins 6 liegende radial nach außen ragende Nocken 6c ist in der in Fig. 3C gezeigten Ausgangsposition noch außerhalb eines Führungsbereichs auf der radialen Innenseite der Verriegelungshülse 8 und wird erst nach dem Einstich in axialer Richtung relativ zur Verriegelungshülse 8 durch eine axiale Verschiebung der Verriegelungshülse 8 relativ zum Gehäuse 2 in einen Eingriffsbereich bewegt, wie in Fig. 3D und in Fig. 4D gezeigt, bei welchem der Nocken 6c mit einer stirnseitigen Abschrägung in axialer Richtung an einer Abschrägung 8b des Steges 8h anliegt. Da in diesem Zustand eine in distale Richtung (in Fig. 3E nach links) wirkende Kraft durch die Nadelschutzhülsenfeder 10 auf die Verriegelungshülse 8 ausgeübt wird, führt die an der Abschrägung 8b vorhandene Anlage an dem gegen axiale Verschiebung durch die Druckkraft der Injektionsfeder 9 gehaltenen Nocken 6c des Klickpins 6 zu einem Drehimpuls der Verriegelungshülse 8 in der durch den Pfeil P gezeigten Richtung. Durch das Verdrehen der Verriegelungshülse 8 relativ zum Gehäuse 2 wird die Verriegelungshülse 8 auch relativ zum Nocken 5c des Mechanikhalters 5 so verdreht, dass der Nocken 5c entweder in die axial verlaufende in Fig. 3E und Fig. 4E gezeigte Verriegelungsbahn 8c eingreift oder kurz vor dem Eingriff steht.

Wird das Gehäuse 2 der Injektionsvorrichtung soweit auf die Injektionsstelle gedrückt, dass die Nadelschutzhülse 3 fast vollständig oder vollständig in das Gehäuse 2 eingeschoben worden ist, wie in Fig. 4A und 4B gezeigt, wird die automatische Injektion ausgelöst. Dabei wird die Verriegelungshülse 8 durch die anliegenden Stege 3a der Nadelschutzhülse 3 innerhalb des Gehäuses 2 axial in proximale Richtung soweit verschoben, dass die Auslöseschnapper 6a das Klick-Pins 6 durch die nicht mehr um die Außennocken 6c liegende und wegverschobene Verriegelungshülse 8 freigegeben werden, wodurch die Auslöseschnapparme 6a radial nach außen ausweichen können. Da die Kolbenstange 7 durch die Injektionsfeder 9 in distale Richtung relativ zum Gehäuse 2 mit einer Kraft beaufschlagt ist, können die Innennocken 6b aus ihrem Eingriff in die Öffnungen 7a der Kolbenstange 7 herausgedrückt werden, was durch die in Fig. 4B gezeigte Abschrägung 6f und 7d unterstützt wird.

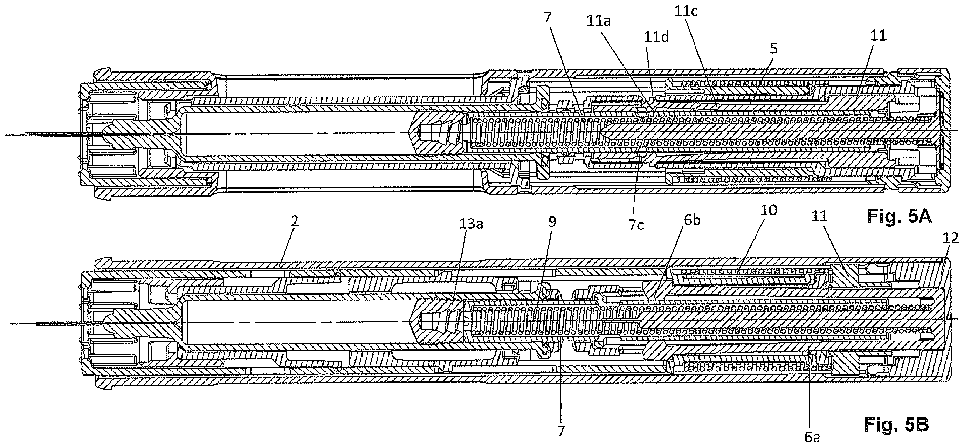

Die entsprechend radial nach außen gedrückten Auslöseschnapparme 6a des Klick-Pins 6 sind in Fig. 5B gezeigt. Hierdurch wird die Kolbenstange 7 freigegeben und kann sich, getrieben durch die Kraft der vorgespannten Injektionsfeder 9, relativ zum Gehäuse 2 innerhalb des Gehäuses 2 in distale Richtung auf den Stopfen 13a bewegen. Des Weiteren wird auch der Klick-Pin 6 freigegeben und durch die Kraft der Injektionsfeder 9 axial innerhalb des Gehäuses 2 in proximale Richtung gedrückt bis die proximale Stirnfläche des Pins 6 auf der distalen Bodenfläche der Endkappe 12 anschlägt, wie in den Figuren 5A und 5B gezeigt. Durch den Aufschlag des Klick-Pins 6 auf der Endkappe 12 wird ein Startsignal oder Startklick erzeugt. Zwischen der Endkappe 12 und dem Klick Pin 6 können Dämpfungselemente angebracht sein, wodurch der Anschlag bzw. Aufprall des Klick Pins 6 verzögert bzw. abgebremst werden kann und so das Startsignal modifiziert werden kann. Dafür kann, wie in den Fig. 3A und 3B gezeigt, zwischen der Endkappe 12 und dem Klick Pin 6 zum Beispiel eine Quetschrippe 12c und eine Gegenrippe 12c' welche in einem Winkel gegenüber der Quetschrippe 12c anliegt angebracht sein. Weiterhin können zusätzliche Dämpfungslaschen oder -Schnapper 12d und gegenüberliegende Dämpfungsrippen 12d'angebracht sein.

### Wie bereits beschrieben wird durch das Aufdrücken der Auslösearme 6a die Nadelschutzverriegelung aktiviert (Fig. 4D)

Die Figuren 5A und 5B zeigen um 90° zueinander versetzte Querschnittsansichten der Injektionsvorrichtung nach erfolgtem Start-Click, welcher den Beginn der Injektion akustisch und taktil signalisiert, und der Aktivierung des nachfolgend beschriebenen Signals zum Anzeigen des Injektionsendes (End-Of-Injection Click).

Die Kolbenstange 7 wird durch die Kraft der sich entspannenden Injektionsfeder 9 in distale Richtung bewegt und gerät in Anlage an den Stopfen 13a, auf welchem die Kolbenstange 7 durch die sich proximal abstützende Injektionsfeder 9 eine in distale Richtung wirkende Kraft ausübt, wodurch der Stopfen 13a in die Spritze 13 eingeschoben wird, um so die in der Spritze 13 enthaltene Substanz zu verdrängen, welche durch die eingestochene Nadel 14 abgeben oder injiziert wird.

Die Kolbenstange 7 ist, wie in Fig. 4A gezeigt, vor dem Verschieben zum Auftreffen auf die proximale Seite des Stopfens 13a noch mit dem Klickelement 11 mittels der Nocken 11a verbunden, welche in korrespondierenden Vertiefungen oder Ausnehmungen der Kolbenstange 7 eingreifen. Dieser Eingriff wird durch den die Klickelementarme 11c umgebenden Mechanikhalter 5 gesichert, welcher verhindert, dass die Klickelementarme 11c radial nach außen ausweichen und so den Eingriff in die Kolbenstange 7 lösen können. Ist die Kolbenstange 7 soweit in distale Richtung verschoben worden, dass Außennocken 11d der Klickelementarme 11c in Ausweichöffnungen des Mechanikhalters 5 eingreifen können, wie in Fig. 5A gezeigt, so werden die Klickelementarme 11c außer Eingriff mit der Kolbenstange 7 gedrängt und weiten sich auf, so dass die verschiebesichere Kopplung mit der Kolbenstange 7 gelöst wird. Hierdurch wird das Klickelement 11 nach hinten gehalten. Da sich die Verriegelungshülsenfeder 10 an ihrem proximalen Ende gegen das Klickelement 11 abstützt, wird die Verriegelungshülsenfeder 10 durch die Verschiebung des Klickelements 11 in distale Richtung gespannt, wie aus den Figuren 5A und 5B ersichtlich. Es erfolgt somit eine Energieübertragung von der sich entspannenden Injektionsfeder 9 auf die sich spannende Verriegelungshülsenfeder 10.

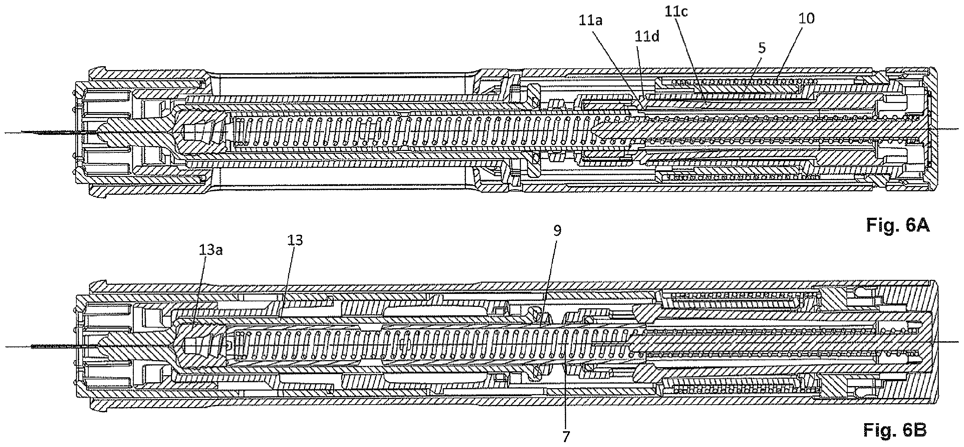

Die Figuren 6A und 6B zeigen um 90° zueinander versetzte Längsquerschnittsansichten der Injektionsvorrichtung nach dem Ausschütten der Substanz. Dabei wurde die nicht mehr zurückgehaltene Kolbenstange 7 durch die Kraft der Injektionsfeder 9 in die Spritze 13 eingeschoben, wodurch der Stopfen 13a in distale Richtung bewegt wurde, bis dieser am Ende des Glaskörpers ansteht und die Substanz somit vollständig ausgeschüttet oder abgegeben wurde.

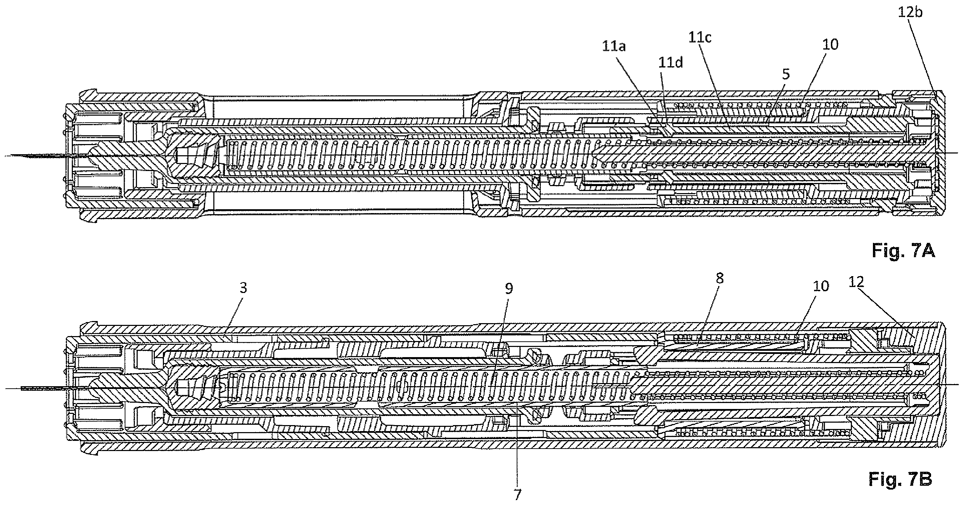

Die eine radial nach innen gerichtete Bewegung der Nocken 11a auf den Klickelementarmen 11c verhindernde Kolbenstange 7, welche an den Innennocken 11c des Klickelements 11 in dem in den Figuren 5A und 5B gezeigten Zustand angelegen ist, ist nach dem Ausschütten, wie in Fig. 6A gezeigt, soweit in distale Richtung verschoben worden, dass die Klickelementarme 11c wieder freigegeben werden. Da das Klickelement 11 durch die Nadelschutzhülsenfeder 10 in proximale Richtung druckbeaufschlagt ist, löst sich der Halteeingriff der Klickelementarme 11c mittels der Außennocken 11d im Mechanikhalter 5 und gibt das Klickelement 11 frei, wie in Fig. 7A gezeigt. Das Klickelement 11 wird nach Freigabe durch die Nadelschutzhülsenfeder 10 in proximale Richtung bewegt bis es am Boden 12b der Endkappe 12 anschlägt und einen Endklick verursacht. Dieser Endklick ist hörbar und von einem Benutzer auch taktil wahrnehmbar, um so das Ende der Injektion zu signalisieren. Der Endklick wird folglich nicht durch die Injektionsfeder 9 bewirkt.

Die Figuren 7A und 7B zeigen um 90° zueinander versetzte Längsquerschnittsansichten der Injektionsvorrichtung bei oder nach erfolgtem Endklick. Wird die Injektionsvorrichtung nach erfolgter Ausschüttung von der Injektionsstelle abgenommen, so bewegt sich die Nadelschutzhülse 3 zusammen mit der auf sie drückenden Verriegelungshülse 8, welche beide durch die Nadelschutzhülsenfeder 10 in distale Richtung mit einer Kraft beaufschlagt werden, nach vorne, also relativ zum Gehäuse 2 axial in distale Richtung.

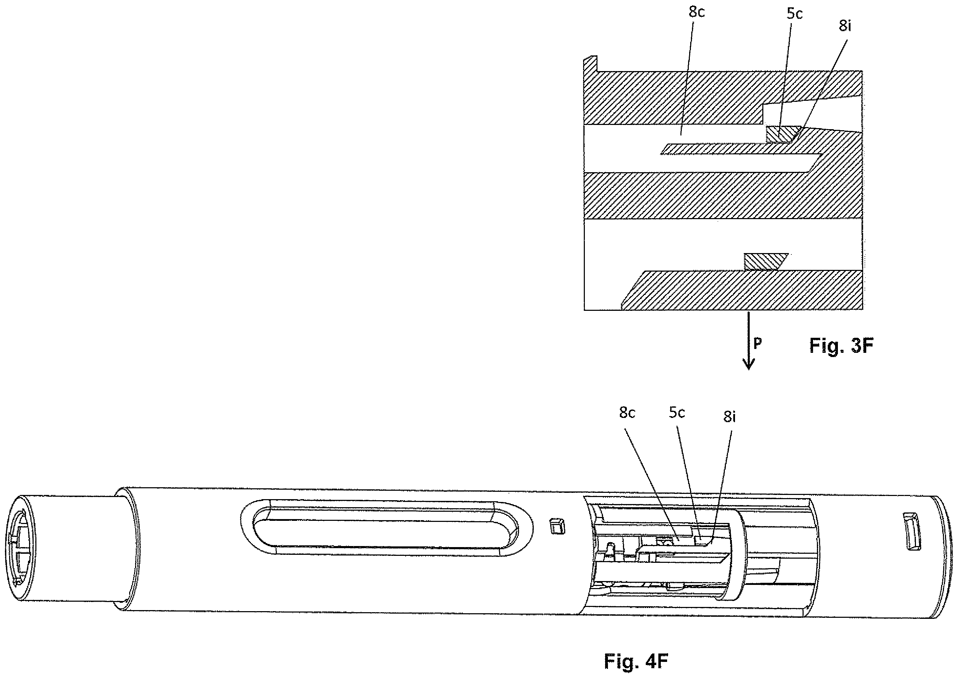

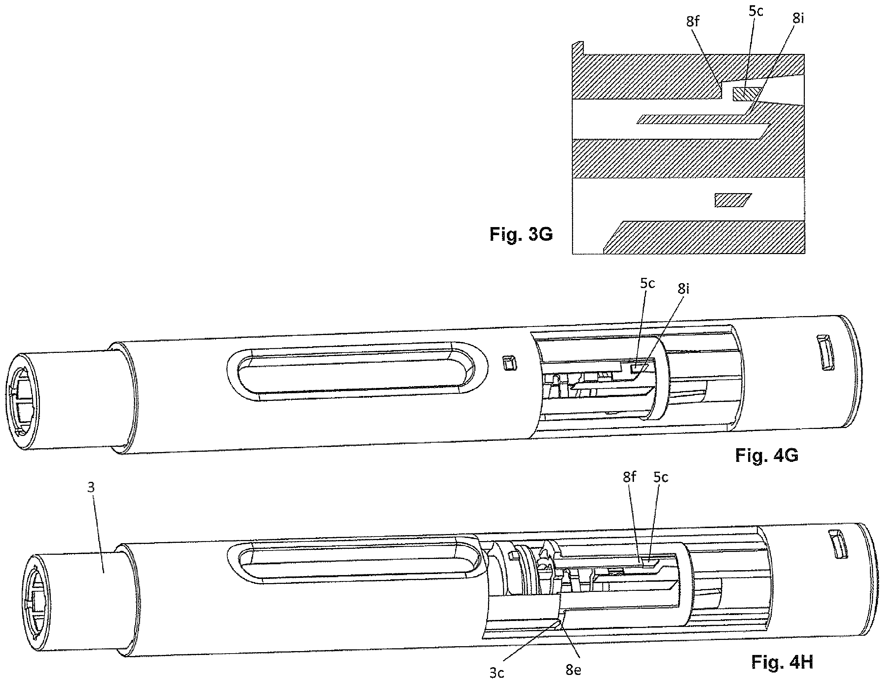

Durch die Kraft der Nadelschutzhülsenfeder 10 wird die Verriegelungshülse 8 in distale Richtung (in Fig. 3E nach links und Fig. 4E) axial verschoben, wobei diese Axialverschiebung eine Drehung der Verriegelungshülse 8 mittels der Abschrägungen 8b und dem Nocken 6c auslöst und dadurch der Nocken 5c in Eingriff mit der axial verlaufenden Nut 8c geführt wird und die Verriegelungshülse 8 somit während dieser Verschiebung verdrehgesichert ist. Ist die Verriegelungshülse 8 soweit verschoben worden, dass der Nocken 5c in Anlage an die Schräge 8i am proximalen Ende der Nut 8c kommt, wie in den Fig. 3F und 4F gezeigt, so führt ein weiterer auf die Verriegelungshülse 8 ausgeübter Druck in distale Richtung zu einer weiteren Drehung der Verriegelungshülse 8 in der durch den Pfeil P gezeigten Richtung, (Fig. 3F, 4F und 3G, 4G) wodurch der Nocken 5c an der Stufe 8f der Verriegelungshülse 8 ansteht und so ein Zurückschieben der Verriegelungshülse 8 und der Nadelschutzhülse 3 in proximale Richtung (in Fig. 4H nach rechts) verhindert.

Ein an einem jeweiligen Steg 3a der Nadelschutzhülse 3 vorgesehener Zentriernocken 3c greift in diesem Zustand in eine Zentriernut 8e der Verriegelungshülse 8 ein und verhindert somit, dass die Verriegelungshülse 8 zurückgedreht werden kann. Somit ist in diesem Zustand die Nadelschutzhülse 3 über die Nadel 14 hinaus ausgeschoben und gegen ein Zurückschieben durch den an der Stufe 8f der Verriegelungsbahn 8c anstehenden Nocken 5c gesichert.

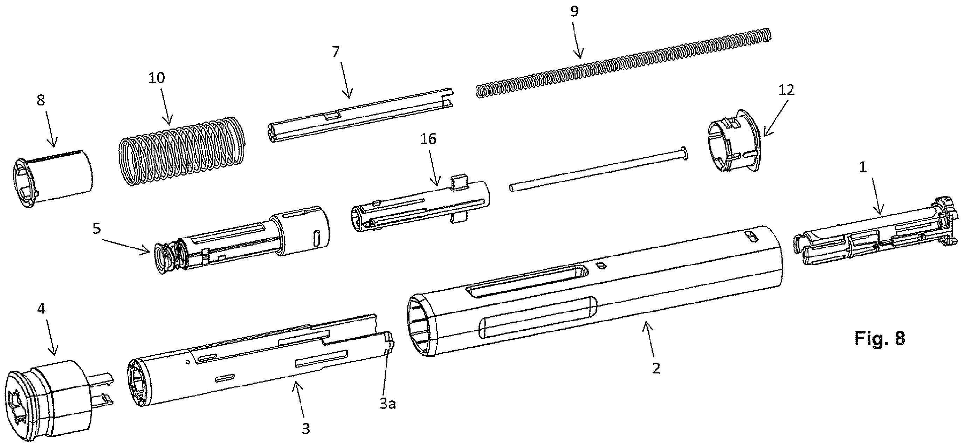

Figur 8 zeigt eine Explosionsansicht einer zweiten Ausführungsform einer Injektionsvorrichtung.

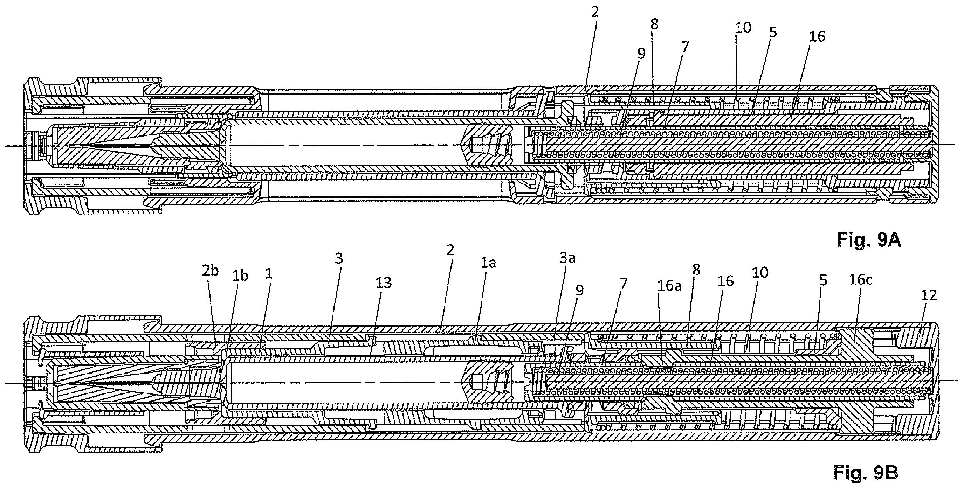

Die Figuren 9A und 9B zeigen um 90° zueinander versetzte Längsquerschnittsansichten im Auslieferungszustand. Die Injektionsfeder 9 ist zwischen Kolbenstange 7 und Endkappe 12 vorgespannt. Eine Freigabehülse 16 liegt nach vorne über Rippen 16c auf dem Mechanikhalter 5 auf. Der Mechanikhalter 5 und die Endkappe 12 sind fest miteinander verbunden. Die Kolbenstange 7 wird in distale Richtung durch einen Auslöseschnapper 16a der Freigabehülse 16 gehalten und mittels der Verriegelungshülse 8 gesichert. Ein Nocken 5c des Mechanikhalters 5 ist mit einer axial verlaufenden Nut 8a der Verriegelungshülse 8 im Eingriff und verhindert ein Verdrehen der Verriegelungshülse 8 relativ zum Mechanikhalter 5.

Die Nadelschutzhülsenfeder 10 ist zwischen der Verriegelungshülse 8 und der Freigabehülse 16 vorgespannt. Der Mechanikhalter 5 und die Endkappe 12 sind gehäusefest und z. B. durch Verschnappen oder Einrasten mit dem Gehäuse 2 verbunden.

Die Verriegelungshülse 8 liegt auf Stegen oder Laschen 3a der Nadelschutzhülse 3 auf, welche nach vorne über einen Nocken 1a auf dem Spritzenhalter 1 gehalten wird. Die Freigabehülse 16 wird nach hinten über die von der Verriegelungshülse 8 gesicherten Auslösearme 16a der Freigabehülse 16 an der Kolbenstange 7 gehalten.

Die Spritze 13 wird ebenso wie in der ersten Ausführungsform beschrieben mittels einer Schulterauflage 1b und mittels eines Ringes bzw. einer Gehäuseverjüngung 2b gesichert.

Die Abnahme der Nadelschutzkappe 4 erfolgt ebenfalls wie im ersten Ausführungsbeispiel beschrieben.

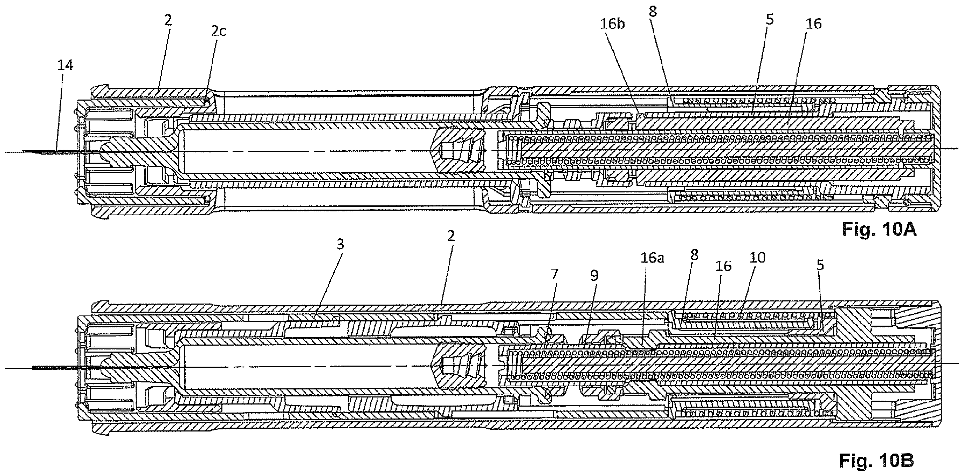

Figuren 10A und 10B zeigen um 90° zueinander verdrehte Längsquerschnittsansichten der Injektionsvorrichtung im eingestochenen Zustand wenn die Injektionsvorrichtung auf die Injektionsstelle gedrückt wird. Die Nadelschutzhülse 3 wird bis auf den Anschlag 2c in das Gehäuse 2 eingedrückt während die Nadel 14 in die Injektionsstelle eingestochen wird. Die Nadelschutzhülse 3 verschiebt die Verriegelungshülse 8 relativ zum Mechanikhalter 5 und der Freigabehülse 16, wodurch die Nadelschutzhülsenfeder 10 gestaucht oder komprimiert wird.

Die Injektion wird durch das vollständige Einschieben der Nadelschutzhülse 3 in das Gehäuse 2 ausgelöst. Dabei wird die Verriegelungshülse 8 relativ zum Gehäuse 2, zum Mechanikhalter 5 und zur Freigabehülse 16 in proximale Richtung (in Fig. 10 nach rechts) verschoben, wobei die Auslöseschnapper 16a freigegeben werden. Durch die Kraft der Injektionsfeder 9 werden die Auslöseschnapper 16a aufgedrückt und gegen die Bewegung der Kolbenstange 7 in nach vorne distale Richtung frei. Die Freigabehülse 16 wird nun über die Schnapper 16b am Mechanikhalter 5 nach hinten gehalten.

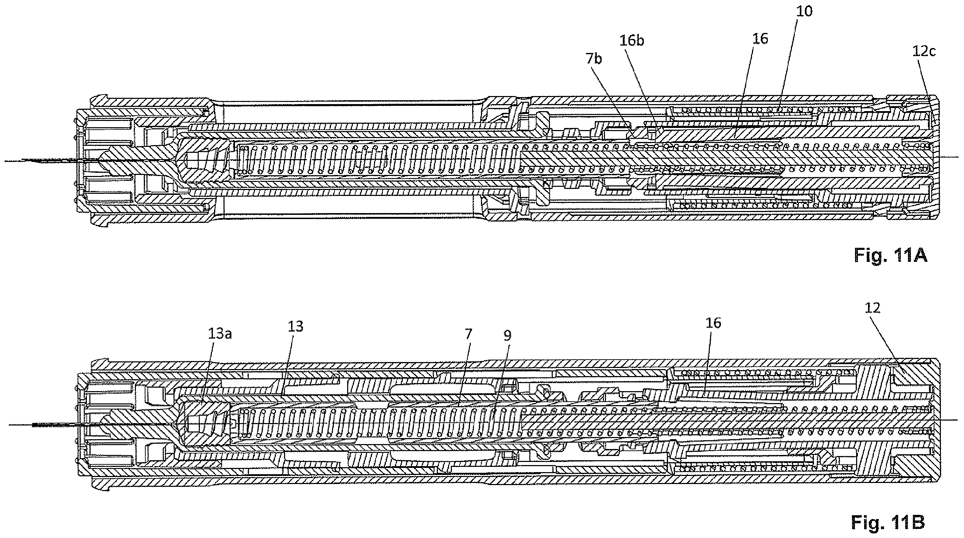

Die Figuren 11A und 11B zeigen um 90° zueinander versetzte Längsquerschnittsansichten der Injektionsvorrichtung nach dem Ausschütten und nach erfolgtem Klick zur Signalisierung des Injektionsendes (End-Of-Injection Click). Dabei ist die Kolbenstange 7 durch die Kraft der Injektionsfeder 9 in distale Richtung bewegt worden, wobei die Kolbenstange 7 auf den Stopfen 13a drückt und diesen in distale Richtung bewegt bis dieser am Ende des Glaskörpers der Spritze 13 ansteht. Die Kolbenstange 7 weist an ihrem proximalen Ende einen Schlitz 7b auf, wodurch die Arme 16b der Freigabehülse 16 am Ende der Injektion freigegeben werden und die Sperrung der Freigabehülse 16 nach hinten aufgehoben wird.

Sobald die Arme 16b der Freigabehülse 16 durch den Schlitz 7b der Kolbenstange 7 freigegeben sind, werden die Arme 16b ausgelenkt, wobei die Freigabehülse 16 durch die Kraft der Nadelschutzhülsenfeder 10 nach hinten bewegt wird und an dem Boden 12c der Endkappe 12 anschlägt und den Endklick verursacht.

Die Nadelschutzverriegelung erfolgt auf gleiche Art wie im ersten Ausführungsbeispiel beschrieben, wobei die ausgelenkten Auslösearme an der Freigabehülse 16 und nicht am Klick-Pin 6 angeordnet sind, so dass diesbezüglich auf obige Beschreibung verwiesen wird.

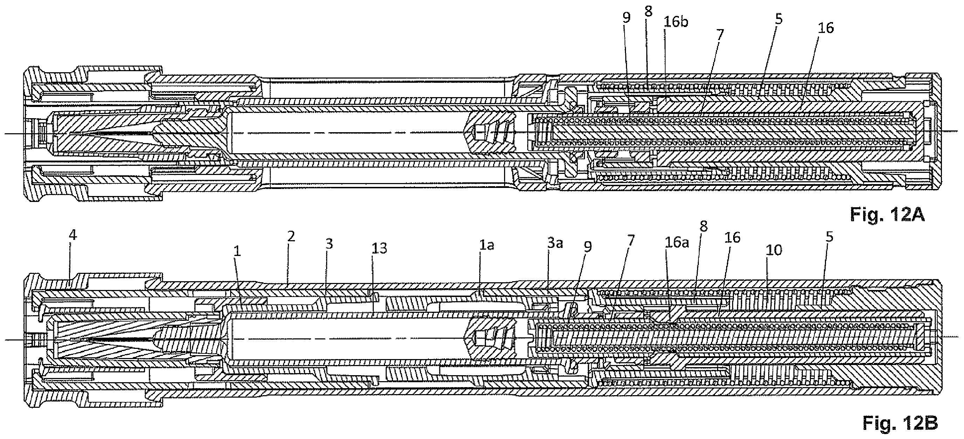

Die Figuren 12A und 12B zeigen um 90° zueinander versetzte Längsquerschnittsansichten einer dritten Ausführungsform einer Injektionsvorrichtung im Auslieferungszustand. Die Injektionsfeder 9 ist zwischen der Kolbenstange 7 und der Freigabehülse 16 vorgespannt. Die Freigabehülse 16 wird durch die Arme 16b am Mechanikhalter 5 gehalten und mittels der Kolbenstange 7 gesichert. Die Kolbenstange 7 wird nach vorne durch die Auslöseschnapper 16a gehalten und mittels der Verriegelungshülse 8 gesichert. Der Nocken 5c des Mechanikhalters 5 ist mit der axial verlaufenden Nut 8a der Verriegelungshülse 8 im Eingriff und verhindert ein Verdrehen der Verriegelungshülse 8 relativ zum gehäusefesten Mechanikhalter 5.

Die Nadelschutzhülsenfeder 10 ist zwischen der Verriegelungshülse 8 und dem Mechanikhalter 5 vorgespannt. Der Mechanikhalter 5 ist mit dem Gehäuse 2 verschnappt. Die Verriegelungshülse 8 liegt auf den Laschen 3a der Nadelschutzhülse 3 auf, welche nach vorne über den Nocken 1 a auf dem Spritzenhalter 1 gehalten wird.

Die Spritze 13 wird wie in den obigen Ausführungsformen beschrieben im Spritzenhalter 1 und Gehäuse 2 gelagert.

Das Abnehmen des Kappenabzugselements 4 zum Abnehmen der Nadelschutzkappe 15 erfolgt wie oben beschrieben.

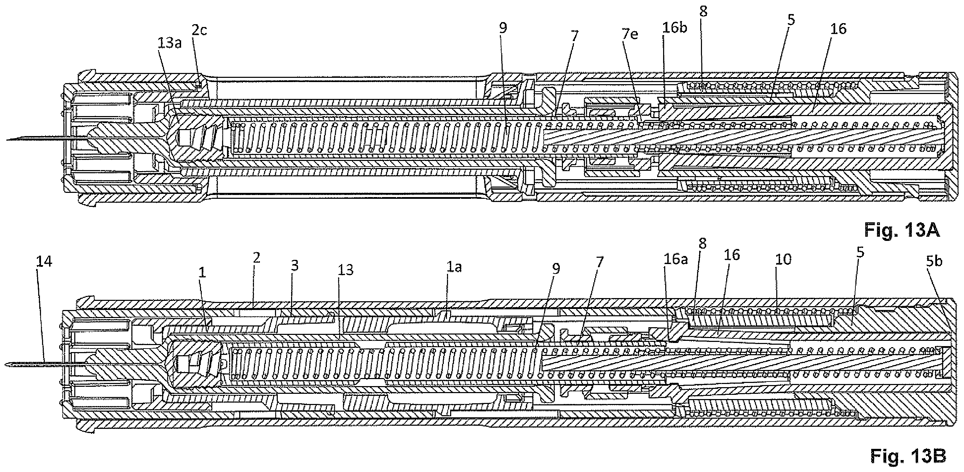

Die Figuren 13A und 13B zeigen um 90° zueinander versetzte Längsquerschnittsansichten der Injektionsvorrichtung, welche auf die Injektionsstelle gedrückt wurde, der Inhalt der Spritze ausgeschüttet wurde und eine Signalisierung des Endes der Injektion erfolgt ist.

Zunächst wird die Nadel 14 eingestochen. Dabei wird die Nadelschutzhülse 3 bis auf den Anschlag 2c in das Gehäuse 2 eingedrückt. Die Nadelschutzhülse 3 verschiebt die Verriegelungshülse 8 relativ zum Mechanikhalter 5 und zur Freigabehülse 16, wobei die Nadelschutzhülsenfeder 10 gestaucht wird.

Die Injektion wird durch das vollständige Einschieben der Nadelschutzhülse 3 in das Gehäuse 2 ausgelöst. Dabei wird die Verriegelungshülse 8 relativ zum Gehäuse 2, zum Mechanikhalter 5 und zur Freigabehülse 16 in proximale Richtung verschoben, wobei die Auslöseschnapper 16a freigegeben werden. Durch die Kraft der Injektionsfeder 9 werden die Auslöseschnapper 16a aufgedrückt und gegen die Bewegung der Kolbenstange 7 nach vorne frei.

Durch die Kraft der Injektionsfeder 9 ist die Kolbenstange 7 in distale Richtung bewegt worden, wobei die Kolbenstange 7 auf den Stopfen 13a drückt und diesen soweit in distale Richtung bewegt bis dieser am Ende des Glaskörpers der Spritze 13 ansteht. Die Kolbenstange 7 weist an ihrem proximalen Ende einen Schlitz 7e auf, wodurch die Arme 16b der Freigabehülse 16 am Ende der Injektion freigegeben werden und die Sperrung der Freigabehülse 16 in proximale Richtung bzw. nach hinten aufgehoben wird.

Sobald die Arme 16b der Freigabehülse 16 durch den Schlitz 7e der Kolbenstange 7 freigegeben sind, werden die Arme 16b ausgelenkt, wobei die Freigabehülse 16 durch die Kraft der Injektionsfeder 9 in proximale Richtung bewegt wird und an dem Boden 5b des Mechanikhalters 5 anschlägt und den Endklick verursacht.

Die Nadelschutzverriegelung erfolgt auf gleiche Art wie im zweiten Ausführungsbeispiel beschrieben.

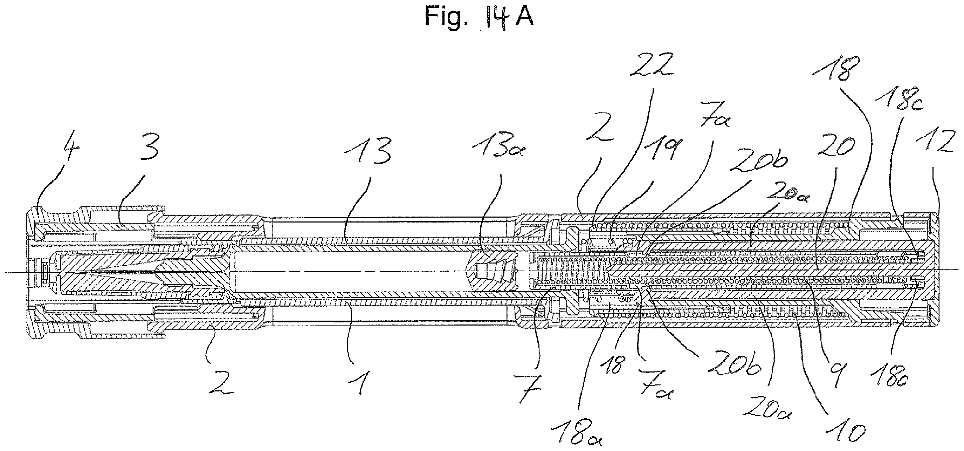

Fig. 14A zeigt eine Längsquerschnittsansicht einer weiteren Ausführungsform einer Injektionsvorrichtung im Auslieferungszustand, bei welcher die Injektionsfeder 9 an ihrem distalen Ende auf der Innenseite der Kolbenstange 7 aufliegt. An ihrem proximalen Ende liegt die Injektionsfeder 9 an einer gehäusefesten Haltehülse 18 an, welche einen Klick-Pin 20 umgibt und radial an einer proximalen Stelle mit Eingriffarmen 18c durch Öffnungen der Kolbenstange 7 in diese eingreift. Der zur Erzeugung des End-Klicks vorgesehene Klick-Pin 20 wird über eine Spritzenfeder 19, welche auf distale Endflächen von Klick-Pin-Annen 20a drückt, in proximale Richtung (in Fig. 14A nach rechts) an die Haltehülse 18 gedrückt, wobei die Endkappe 12 ein Teil der Haltehülse 18 sein kann. Die Spritzenfeder 19 stützt sich in proximaler Richtung an den Armen 20a des Klick-Pins 20 ab und drückt in distale Richtung auf die Spritze 13, um diese durch den so erzeugten in distale Richtung wirkenden Druck sicher im Spritzenhalter 1 zu lagern. Die Arme 20a des Klick-Pins 20 sind mittels radial nach innen vorstehender Nocken 20b im Eingriff in Öffnungen 7a der Kolbenstange 7 und können bei einer distal gerichteten Bewegung der Kolbenstange 7 mitgenommen werden, so dass die Kolbenstange 7 bei einer distalen Bewegung aus dem in Fig. 14A gezeigten Auslieferungszustand den Klick-Pin 20 zunächst mitnimmt. Ein Lösen der Halteverbindung 7a, 20b durch radiales Ausweichen der Arme 20a wird durch die den Klick-Pin 20 umgebende Haltehülse 18 verhindert.

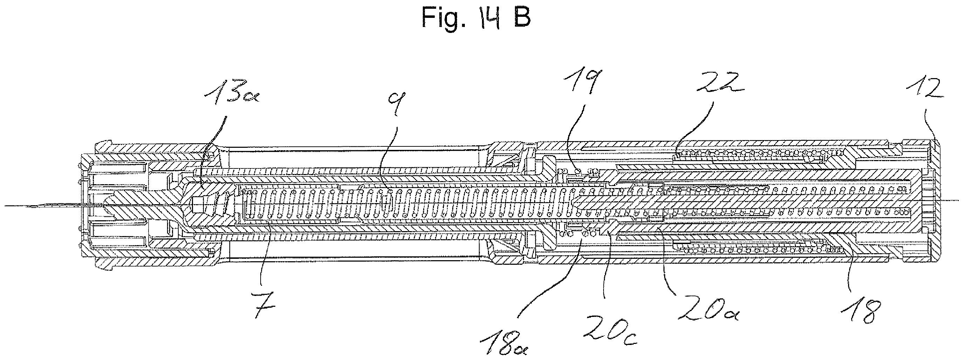

Wird das Gehäuse 2 der Injektionsvorrichtung so weit auf eine Injektionsstelle gedrückt, dass die Nadelschutzhülse 3 fast vollständig oder vollständig in das Gehäuse 2 eingeschoben worden ist, wie in Fig. 14B gezeigt, wird die automatische Injektion ausgelöst.

Dabei wird eine Auslösehülse 22 durch die anliegenden Stege 3a der Nadelschutzhülse 3 gegen die Kraft der Nadelschutzhülsenfeder 10 innerhalb des Gehäuses 2 axial in proximale Richtung soweit verschoben, dass ein Halteelement der Haltehülse 18, welches eingreift in die Kolbenstange 7, freigegeben wird.

Da die Kolbenstange 7 durch die Injektionsfeder 9 in distale Richtung relativ zum Gehäuse 2 mit einer Kraft beaufschlagt ist, kann die nun freigegebene Kolbenstange 7 in distale Richtung auf den Stopfen 13a der Spritze 13 bewegt werden und diesen Stopfen 13a in die Spritze 13 einschieben, um so die Ausschüttung durchzuführen. Während der Ausschüttung wird der Klick-Pin 20 durch die Kolbenstange 7 mitgenommen, bis die Arme 20a des Klick-Pins in der Freistellung 18a der Haltehülse 18 radial auslenken können und durch diesen Eingriff der radial an den Armen 20a außenliegenden Nocken 20c an der Haltehülse 18 gehalten werden. Ein radial nach innen gerichtetes Ausweichen der Arme 20a des Klick-Pins 20 ist durch die innen an den Nocken 20b anliegende Kolbenstange 7 so lange unmöglich, bis die Kolbenstange 7 soweit in distale Richtung bewegt wurde, dass die vollständige Ausschüttung erfolgt ist.

Am Ende der Ausschüttung können die Arme 20a des Klick-Pins 20 radial nach innen ausweichen, da die Kolbenstange 7 soweit in distale Richtung bewegt wurde, dass entweder gemäß einer nicht gezeigten Ausführungsform die Kolbenstange schon vollständig an den Nocken 20b vorbeibewegt wurde oder, wie in Fig. 14B gezeigt, die innen liegenden Nocken 20b in Öffnungen oder Freistellungen an der Kolbenstange 7 zurück lenken können. Der Klick-Pin 20 wird somit nicht mehr durch Nocken 20c oder 20b gehalten und durch die vorspannte Spritzenfeder 19 in proximale Richtung beschleunigt, um an der Haltehülse 18 oder alternativ an der Endkappe 12 (nicht gezeigt) anzuschlagen und so ein End-Klick-Geräusch zu erzeugen.

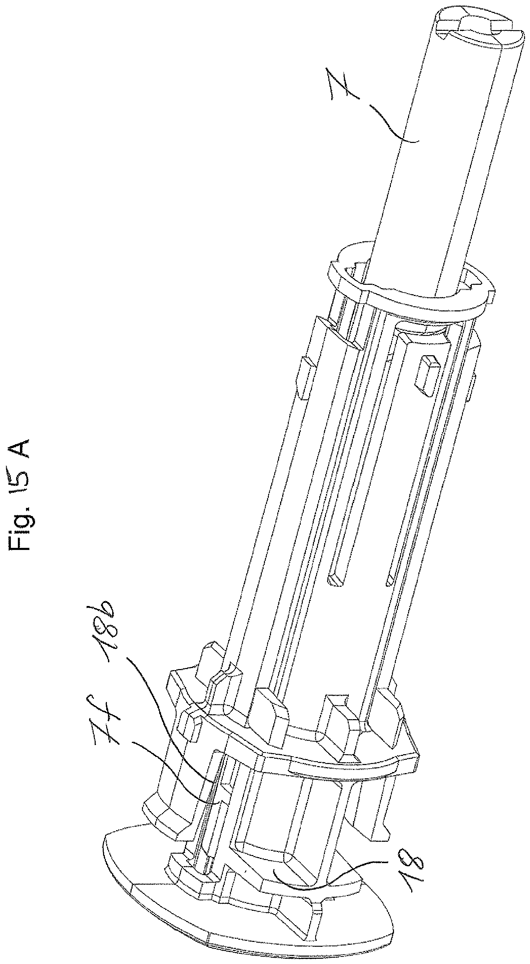

Fig. 15A zeigt eine weitere Ausführungsform einer Funktionseinheit zur Erzeugung eines End-Klicks. Dabei ist eine Injektionsfeder zwischen der Kolbenstange 7 und einen Stabilisierungs-Pin oder der Haltehülse 18 auf Torsion vorgespannt. Der Stabilisierungs-Pin liegt auf dem Boden der Haltehülse 18 auf und verläuft axial innerhalb der Injektionsfeder und ist mit der Haltehülse 18 gegen ein Verdrehen gesichert.

Die Kolbenstange 7 ist mittels einer Linearführungselements 7f in der Haltehülse 18 in einer Linearführung 18b linear geführt.

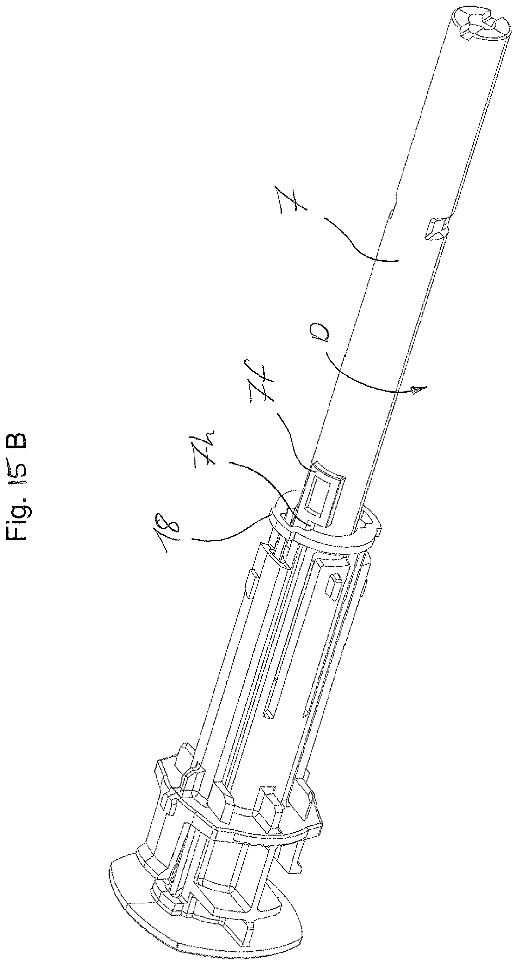

Nach erfolgter Injektion durch axial nach vorne gerichtetes Ausschieben der Kolbenstange 7 aus der Haltehülse 18, wie in Fig. 15B gezeigt, wird die Kolbenstange 7 durch das Herausfahren des Linearführungselements 7f aus der Linearführung 18b entkoppelt. Durch das Torsionsmoment der Injektionsfeder 9 wird die Kolbenstange 7 in der durch den die Drehrichtung D angebenden Pfeil definierten Richtung gedreht und mit einem Anschlagelement 7h in Umfangsrichtung gegen die Haltehülse 18 oder eine Linearführungsnutinnenfläche geschlagen, wodurch ein End-Klick erzeugt werden kann.

Dabei kann optional auch die Kolbenstange 7 auf dem Stopfen 13a der Spritze 13 drehen.

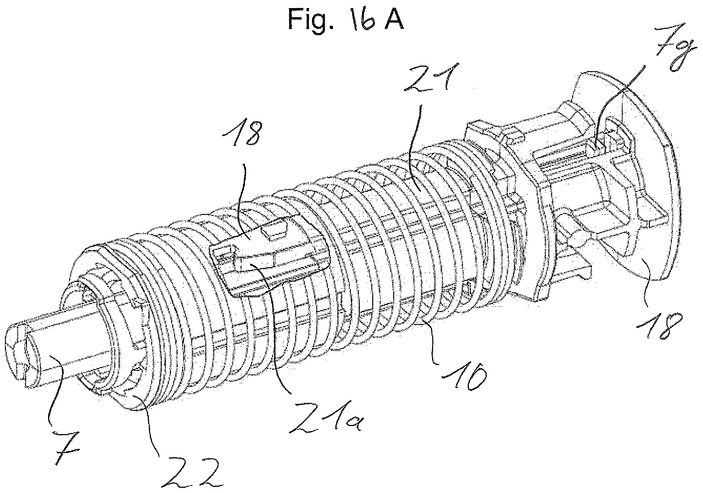

Fig. 16A zeigt eine weitere Ausführungsform einer Funktionseinheit zur Erzeugung eines Endklicks, wobei die Energie für den End-Klick durch die Nadelschutzhülsenfeder 10 zur Verfügung gestellt wird. Die Nadelschutzhülsenfeder 10 ist zwischen der in Fig. 16A Auslösehülse 22 und der Klick-Hülse 21 vorgespannt. Die Klick-Hülse 21 weist Haken 21a auf, welche in die Haltehülse 18 eingreifen.

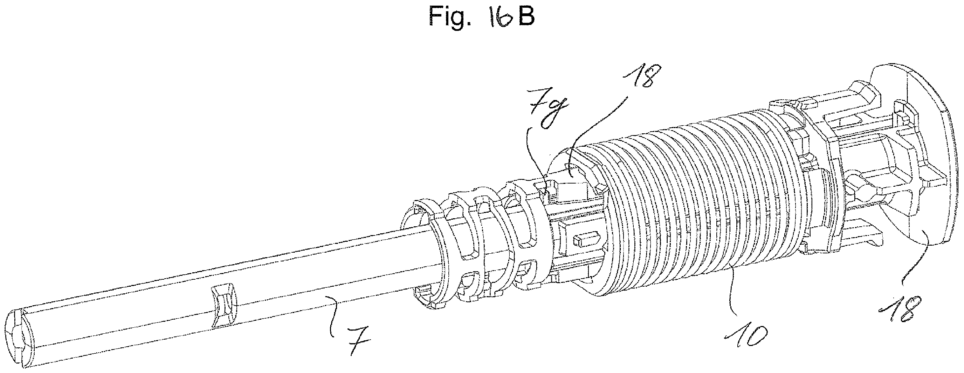

Die Kolbenstange 7 weist einen Nocken 7g auf, welcher gegen Ende der Injektion, wie in Fig. 16B gezeigt, durch ein Vorbeifahren von proximal nach distal (in Fig. 16B von rechts nach links) die Haken 21a der Klick-Hülse 21 aufdrückt, wodurch diese aus der Haltehülse 18 aushängen und so die Kopplung der Klick-Hülse 21 mit der Haltehülse 18 gelöst wird.

Die Klick-Hülse 21 wird durch die Nadelschutzhülsenfeder 10 nach hinten bzw. in proximale Richtung zu einem Anschlag auf der Haltehülse 18 beschleunigt oder bewegt, wodurch der End-Klick erzeugt wird.

### Bezugszeichen:

- 1: Spritzenhalter
- 1a: proximaler Außennocken
- 1b: Vorsprung/Schulterauflage
- 1c: distaler Außennocken
- 1d: Federarm
- 2: Gehäuse
- 2b: Gehäuseverjüngung
- 2c: Anschlag
- 2e: Rippen
- 2f: Rippen
- 3: Nadelschutzhülse
- 3a: Stege
- 3b: Schnapphalterung
- 3c: Zentriernocken
- 3d: Durchtrittsöffnung
- 3e: Axialnut
- 4: Kappenabzugselement
- 4a: Schnapphaken
- 4b: Schnappelement
- 5: Mechanikhalter
- 5b: Boden
- 5c: Nocken
- 6: Klick-Pin (Start-Klick)
- 6a: Auslöseschnapparme
- 6b: Innennocken
- 6c: Außennocken
- 6d: tellerförmiges Bodenelement
- 6e: Mittelsteg
- 6f: Abschrägung
- 7: Kolbenstange
- 7a: Öffnung
- 7b: Bodenelement
- 7c: Öffnung
- 7d: Abschrägung
- 7e: Schlitz
- 7f: Linearführungselement
- 7g: Nocken
- 7h: Anschlagelement
- 8: Verriegelungshülse
- 8a: Axialnut
- 8b: Auslösekurve/Schräge
- 8c: Verriegelungsbahn
- 8e: Zentriernut
- 8f: Stufe
- 8g: Abstützflansch
- 8h: Steg
- 8i: Schräge
- 9: Injektionsfeder
- 10: Nadelschutzhülsenfeder
- 11: End-Klickelement
- 11a: Nocken
- 11b: Aufweitung
- 11c: Federarme
- 11d: Außennocken
- 12: Endkappe
- 12a: Schnapper
- 12b: tellerförmiger Boden
- 13: Spritze
- 13a: Stopfen
- 13b: Substanzaufnahmeraum
- 14: Nadel
- 15: Nadelschutzkappe
- 15a: Gumminadelschutzelement
- 16: Freigabehülse
- 16a: Auslösearme
- 16b: Schnapparme
- 17: Führungspin
- 18: Haltehülse
- 18a: Freistellung
- 18b: Linearführung
- 18c: Eingriffarm
- 19: Spritzenfeder
- 20: Klick-Pin (End-Klick)
- 20a: Arme
- 20b: Nocken (radial innen)
- 20c: Nocken (radial außen)
- 21: Klick-Hülse
- 21a: Haken
- 22: Auslösehülse

## Patentansprüche

1. Vorrichtung zum Verabreichen einer Substanz mit:
1.1 einer Nadel, durch welche die Substanz abgegeben werden kann;
1.2 einem verschiebbaren Nadelschutzelement (3) für die Nadel (14);
1.3 einer Nadelschutzhülsenfeder (10), welche mit dem Nadelschutzelement (3) gekoppelt ist; und
1.4 einem Anschlagelement (11), welches ein Signal erzeugen kann, wenn oder nachdem eine vorgegebene oder die gesamte Menge der abzugebenden Substanz abgegeben wurde; wobei
1.5 das Anschlagelement (11) mit der Nadelschutzhülsenfeder (10) gekoppelt ist und für die Erzeugung des Signals von der Nadelschutzhülsenfeder (10) über eine vorgegebene und entlang einer geraden Strecke beschleunigt wird, um einen Aufschlag und somit das bemerkbare Signal zu erzeugen, wobei die Nadelschutzhülsenfeder (10) das Nadelschutzelement (3) mit Druck beaufschlagt oder Kraft auf dieses ausübt, um das Nadelschutzelement (3) relativ zur Nadel in eine Schutzposition zu bringen oder zu halten; **gekennzeichnet durch**
1.6 eine Injektionsfeder (9), welche die Energie zur Bewirkung der Substanzabgabe zur Verfügung stellt, wobei die Injektionsfeder (9) Energie an die Nadelschutzhülsenfeder (10) während der Substanzabgabe abgibt.

2. Vorrichtung nach Anspruch 1 mit einem Rückkopplungselement (6), welches den Beginn einer Substanzabgabe signalisieren kann.

3. Vorrichtung nach Anspruch 2, wobei das Rückkopplungselement (6) von der Injektionsfeder (9) angetrieben wird.

4. Vorrichtung nach einem der vorhergehenden Ansprüche, wobei das Anschlagelement (11) an einem Gehäuse (2) der Vorrichtung oder einem gehäusefesten Element (5, 12) anschlägt.

5. Vorrichtung nach Anspruch 2, mit einem Dämpfungselement oder Modulationselement, mittels welchem die Rückkopplung durch das Rückkopplungselement (6) modifiziert oder gebremst werden kann.

6. Vorrichtung nach einem der vorhergehenden Ansprüche, wobei das Anschlagelement (11) ein akustisches und/oder taktiles Signal erzeugen kann.

7. Vorrichtung nach einem der vorhergehenden Ansprüche, wobei das Nadelschutzelement (3) die Nadel (14) vor und/oder nach der Substanzabgabe abdeckt oder umgibt.

8. Vorrichtung nach einem der vorhergehenden Ansprüche mit einer lösbaren Haltevorrichtung, um das Anschlagelement (11) bis zum Ende der Substanzabgabe zu halten.

9. Vorrichtung nach Anspruch 7, wobei das Nadelschutzelement (3) gegenüber der Nadel (14) in eine proximale Richtung verschiebbar ist, um zumindest einen distalen Bereich der Nadel (14) freizugeben.

10. Vorrichtung nach einem der vorhergehenden Ansprüche, umfassend einen unbeweglich in einem Gehäuse (2) der Vorrichtung angeordneten Spritzenhalter (1) zur Aufnahme der Spritze (13).

## Claims

1. Device for administering a substance, comprising:
1.1 a needle, through which the substance can be delivered;
1.2 a movable needle protection element (3) for the needle (14);
1.3 a needle protection sleeve spring (10), which is coupled to the needle protection element (3); and
1.4 a stop element (11), which can generate a signal when or after a predetermined or the entire amount of the substance to be delivered has been delivered;
1.5 the stop element (11) being coupled to the needle protection sleeve spring (10) and, to generate the signal, accelerated over a predetermined and along a straight path by the needle protection sleeve spring (10) in order to generate an impact and thus the noticeable signal, the needle protection sleeve spring (10) applying pressure to the needle protection element (3) or exerting force on it in order to bring or hold the needle protection element (3) in a protective position relative to the needle; **characterized by**
1.6 an injection spring (9), which provides the energy to effect the substance delivery, the injection spring (9) delivering energy to the needle protection sleeve spring (10) during the substance delivery.

2. Device according to claim 1, with a feedback element (6), which can signal the start of a substance delivery.

3. Device according to claim 2, wherein the feedback element (6) is driven by the injection spring (9).

4. Device according to any of the preceding claims, wherein the stop element (11) strikes a housing (2) of the device or an element (5, 12) fixed to the housing.

5. Device according to claim 2, with a damping element or modulation element, by means of which the feedback by the feedback element (6) can be modified or braked.

6. Device according to any of the preceding claims, wherein the stop element (11) can generate an acoustic and/or tactile signal.

7. Device according to any of the preceding claims, wherein the needle protection element (3) covers or surrounds the needle (14) before and/or after the substance delivery.

8. Device according to any of the preceding claims, with a releasable holding device to hold the stop element (11) until the end of the substance delivery.

9. Device according to claim 7, wherein the needle protection element (3) is movable relative to the needle (14) in a proximal direction in order to expose at least a distal region of the needle (14).

10. Device according to any of the preceding claims, comprising a syringe holder (1) arranged immovably in a housing (2) of the device for receiving the syringe (13).

## Revendications

1. Dispositif permettant l'administration d'une substance comportant :
1.1 une aiguille à travers laquelle la substance peut être distribuée ;
1.2 un élément de protection d'aiguille (3) pouvant être coulissé pour l'aiguille (14) ;
1.3 un ressort de douille de protection d'aiguille (10) accouplé à l'élément de protection d'aiguille (3) ; et
1.4 un élément de butée (11) pouvant générer un signal lorsqu'une quantité prédéfinie ou que la quantité totale de la substance à distribuer a été distribuée ou après qu'elle l'a été ; dans lequel
1.5 l'élément de butée (11) est accouplé au ressort de douille de protection d'aiguille (10) et, pour la génération du signal, est accéléré par le ressort de douille de protection d'aiguille (10) le long d'une ligne droite prédéfinie afin de générer un impact et ainsi le signal détectable, dans lequel le ressort de douille de protection d'aiguille (10) exerce une pression ou une force sur l'élément de protection d'aiguille (3) afin d'amener ou de maintenir l'élément de protection d'aiguille (3) dans une position de protection par rapport à l'aiguille ; **caractérisé par**
1.6 un ressort d'injection (9) qui fournit l'énergie permettant de provoquer la distribution de substance et la rendre disponible, dans lequel le ressort d'injection (9) distribue de l'énergie au ressort de douille de protection d'aiguille (10) pendant la distribution de substance.

2. Dispositif selon la revendication 1, comportant un élément de rétroaction (6) pouvant signaler le début d'une distribution de substance.

3. Dispositif selon la revendication 2, dans lequel l'élément de rétroaction (6) est propulsé par le ressort d'injection (9).

4. Dispositif selon l'une des revendications précédentes, dans lequel l'élément de butée (11) vient en butée contre un boîtier (2) du dispositif ou un élément (5, 12) fixe par rapport au boîtier.

5. Dispositif selon la revendication 2, comportant un élément d'amortissement ou un élément de modulation au moyen duquel la rétroaction peut être modifiée ou freinée par l'élément de rétroaction (6).

6. Dispositif selon l'une des revendications précédentes, dans lequel l'élément de butée (11) génère un signal acoustique et/ou tactile.

7. Dispositif selon l'une des revendications précédentes, dans lequel l'élément de protection d'aiguille (3) recouvre ou entoure l'aiguille (14) avant et/ou après la distribution de substance.

8. Dispositif selon l'une des revendications précédentes, comportant un dispositif de maintien amovible afin de maintenir l'élément de butée (11) jusqu'à la fin de la distribution de substance.

9. Dispositif selon la revendication 7, dans lequel l'élément de protection d'aiguille (3) peut être coulissé par rapport à l'aiguille (14) dans une direction proximale afin de libérer au moins une zone distale de l'aiguille (14).

10. Dispositif selon l'une des revendications précédentes, comprenant un support de seringue (1) disposé de manière immobile dans un boîtier (2) du dispositif permettant de recevoir la seringue (13).
